# EUROPEAN PATENT SPECIFICATION

(11) **EP 2 639 293 B1**
(45) Date of publication and mention of the grant of the patent: **20.10.2021**
(21) Application number: 11839327.1
(22) Date of filing: 10.11.2011
(51) Int. Cl.: C12M 3/00, C12N 5/00, C12M 1/12

(54) **CELL CULTURE CHAMBER, METHOD FOR PRODUCING SAME, TISSUE MODEL USING CELL CULTURE CHAMBER, AND METHOD FOR PRODUCING SAME**
ZELLKULTURKAMMER, HERSTELLUNGSVERFAHREN DAFÜR, GEWEBEMODELL MIT DER ZELLKULTURKAMMER UND HERSTELLUNGSVERFAHREN DAFÜR
CHAMBRE DE CULTURE CELLULAIRE, SON PROCÉDÉ DE PRODUCTION, MODÈLE DE TISSU UTILISANT LA CHAMBRE DE CULTURE CELLULAIRE ET SON PROCÉDÉ DE PRODUCTION

(30) Priority: 12.11.2010 JP 2010254255
(43) Date of publication of application: 18.09.2013
(73) Proprietor: Kanto Kagaku Kabushiki Kaisha, Tokyo 103-0023 (JP)
(72) Inventor: TAKEZAWA Toshiaki, Machida-shi Tokyo 194-0211 (JP); YAMAGUCHI Hiroyuki, Isehara-shi Kanagawa 259-1146 (JP); KUROYAMA Hiroyuki, Isehara-shi Kanagawa 259-1146 (JP); SAWAGUCHI Tomoya, Isehara-shi Kanagawa 259-1146 (JP)
(74) Representative: Dehns
(86) International application number: PCT/JP2011/076009
(87) International publication number: WO 2012/063925

(56) References cited:
- WO-A1-2005/014774
- WO-A1-2005/014774
- WO-A1-2007/032224
- WO-A2-2007/114897
- JP-A- 8 228 768
- JP-A- 2007 167 002
- JP-A- 2007 185 107
- JP-A- 2007 204 881
- US-A1- 2002 086 423
- TOSHIAKI TAKEZAWA ET AL.: 'Atarashii Biomaterial: Toriatsukaisei ni Sugureta Collagen Vitrigel-maku Kansotai' REGENERATIVE MEDICINE vol. 10, 01 February 2011, page 265, 2P-099, XP008171358
- TAKEZAWA ET AL.: 'Collagen Vitrigel: A Novel Scaffold That Can Facilitate a Three- Dimensional Culture for Reconstructing Organoids' CELL TRANSPLANTATION vol. 13, 2004, pages 463 - 473, XP003003826

## Description

### Technical Field

The present invention relates to cell culture chambers provided with a dried vitrigel membrane, methods for producing such chambers, tissue models using the cell culture chambers, and methods for producing the same.

### Background Art

In the studies of drug discovery and alternative methods for animal experiment, there have been ongoing demands for the development of a culture system that can be used to easily construct a three-dimensional tissue model that reflects an organism with the use of a variety of functional cells. The three-dimensional culture technique that uses a collagen gel as a culture support of cells is particularly useful for the reconstruction of models such as an angiogenesis model, a cancer infiltration model, and an epithelium mesenchyme model. The technique, however, is insufficient, and has not been used extensively.

Possible reasons for this include difficulties in handling the conventional collagen gels because of the softness of the gels attributed to the constituent low-density fibers, and the nontransparent nature of the gels, which often makes the phase-contrast microscopy of the cultured cells difficult.

As a countermeasure against these problems, the present inventors have established a technique for converting the physical properties of a collagen gel into a thin membrane of excellent strength and excellent transparency with good reproducibility. This is achieved by injecting a collagen sol into a culture Petri dish at low temperature after imparting optimum salt concentration and hydrogen ion concentration (pH) for the gelation of the collagen sol. The collagen sol is then maintained at optimum temperature to gelate, and sufficiently dried at low temperature to vitrify via the gradual removal of not only the free water but the bonding water. The gel is then rehydrated (PTL 1).

In the case of hydrogel, gel components other than collagen also can be vitrified and then rehydrated to convert the gel into a stable, new physical property state. The gel produced via the vitrifying step and having such a new physical property state has been termed the "vitrigel" (NPL 1).

The thin collagen vitrigel membranes that have been developed to date are several ten micrometer-thick transparent thin membranes of intertwining high-density collagen fibers comparative to the connective tissues of the body, and have excellent protein permeability and excellent strength. Further, because various substances can be added to the collagen sol during the fabrication, the properties of the additional substances can be reflected in the thin collagen vitrigel membrane. Further, for example, the thin collagen vitrigel membrane, when embedded with a ring-shaped nylon membrane support, can easily be handled with tweezers.

The present inventors further developed the thin collagen vitrigel membrane technique, and proposed a technique for improving the transparency of the thin collagen vitrigel membrane and the reproducibility of film production (PTL 2, which describes a cell culture carrier comprising a hydrated thin film), a technique for producing a filamentous or tubular collagen vitrigel instead of a film shape (PTL 3), a technique for securing or moving the collagen vitrigel by using magnetism (PTL 4), and the like .

### Citation List

### Patent Documents

Patent Document 1: JP-A-8-228768 (PTL1)
Patent Document 2: WO2005/014774 (PTL2)
Patent Document 3: JP-A-2007-204881 (PTL3)
Patent Document 4: JP-A-2007-185107 (PTL4)

### Non Patent Documents

Non Patent Document 1: Takezawa T, et al., Cell Transplant. 13: 463-473, 2004 (NPL1)
Non Patent Document 2: Takezawa T, et al., J. Biotechnol. 131: 76-83, 2007 (NPL2)

### Summary of Invention

### Problem To Be Solved by Invention

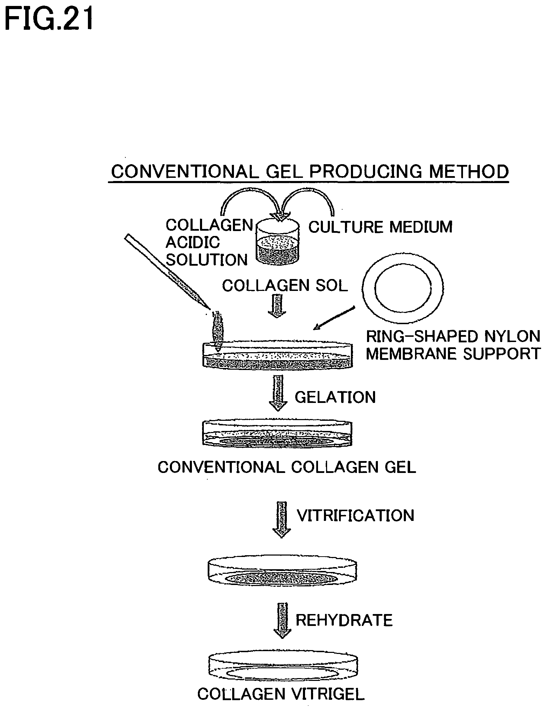

In conventional thin collagen vitrigel membrane producing methods, for example, as shown in FIG. 21, a collagen sol is injected into a plastic culture Petri dish in a predetermined amount to obtain a thin collagen vitrigel membrane of an arbitrary thickness. Following gelation, the gel is dried to vitrify, and rehydrated.

It is therefore not possible with conventional producing methods to detach the dried thin collagen vitrigel membrane from the culture Petri dish, and the dried thin collagen vitrigel membrane produced is adhering to the bottom and wall surfaces of the culture Petri dish. The dried thin collagen vitrigel membrane thus cannot be freely handled in the membrane state, and cannot be cut into an arbitrary fine shape.

Under these circumstances, the present inventors invented a quick method of the mass production of a dried vitrigel membrane that can be shaped as desired and has excellent ease of handling. (Japanese patent application 2010-188887).

The method can be used to obtain a dried vitrigel membrane in the membrane state without the membrane being adhered to the culture Petri dish. By taking advantage of the good ease of handling and processibility, the method has potential to establish a novel use of the dried vitrigel membrane not possible with the conventional methods.

For the development of pharmaceutical preparations and cosmetics and home chemical goods such as detergents, it has been common practice to evaluate the efficacy and toxicity of the raw material chemicals against the target living organism. Conventionally, such evaluations have been performed by conducting cell culture experiments, in which cells derived from humans and animals are two-dimensionally cultured in plane culture, and exposed to chemicals to assess their effects. In the case where the two-dimensional cell culture experiment fails to sufficiently evaluate the efficacy and toxicity of interest, animal experiments are conducted by administering chemicals to animals such as mice, rats, rabbits, dogs, and monkeys to examine their effects. However, in keeping with the increased consciousness of animal protection and for cost considerations, biochemical evaluation, molecular biological evaluation, and tissue pathological evaluation using a three-dimensional tissue model reflective of a biological tissue, particularly organ units such as epithelium and mesenchyme have attracted interest. Further, from the standpoint of extrapolating the ADMET
(absorption-distribution-metaboiism-excretion-toxicity) of chemicals against humans, there is a growing interest in the evaluation system that uses human cells to overcome the problem of species difference. To date, there have been developed a variety of three-dimensional tissue models reconstructed from human cells by tissue engineering. However, as it currently stands, not all techniques proposed so far necessarily reconstruct a three-dimensional tissue model that can predict the chemical ADMET against all organs.

The present invention has been made under these circumstances, and it is an object of the present invention to provide a cell culture chamber that uses a dried vitrigel membrane that can be shaped as desired and has excellent ease of handling, and a three-dimensional tissue model that can be used for the chemical ADMET evaluation or other purposes with the cell culture chamber.

### Means for Solving the Problem

In order to solve the foregoing problems, the present invention provides the following cell culture chambers and tissue models, among others.
<1> A single cell-culture chamber comprising a dried vitrigel membrane covering and secured to one open end surface of a tubular frame, wherein the dried vitrigel membrane is secured to the open end surface of the frame:
   with a urethane-based adhesive; or
   with double-sided tape; or
   by heat welding.
<2> The single cell-culture chamber, wherein the dried vitrigel membrane has substantially the same shape as the open end surface of the frame.
<3> The single cell-culture chamber, wherein the frame includes an outwardly protruding stopper provided on an outer periphery portion of the open end surface opposite from the end surface covered by and secured to the dried vitrigel membrane.
<4> A double cell-culture chamber comprising two tubular frames joined and secured to each other with a dried vitrigel membrane interposed between the opposing open end surfaces of the tubular frames so as to form a first chamber and a second chamber via the dried vitrigel membrane, wherein the dried vitrigel membrane is secured to the open end surface of the frame:
   with a urethane-based adhesive; or
   with double-sided tape; or
   by heat welding.
<5> The double cell-culture chamber, wherein the dried vitrigel membrane has substantially the same shape as the open end surfaces of the frames.
<6> The double cell-culture chamber, wherein the two frames are adhesively secured to each other from outside with a film-shaped adhesive material around the opposing open end surfaces.
<7> A tissue model constructed from a single- or multi-layer culture of cells seeded on the dried vitrigel membrane inside the single cell-culture chamber.
<8> The tissue model of <7>, wherein the tissue model is any one of a chemical transdermal absorption model, a chemical corneal permeation model, a chemical gastrointestinal absorption model such as in intestinal tract, a chemical airway absorption model such as in lungs, a chemical vascular permeation model, a chemical hepatic metabolism model, a chemical renal glomerular filtration and excretion model, a chemical dermotoxicity evaluation model, a chemical keratotoxicity evaluation model, a chemical oral mucosal toxicity evaluation model, a chemical neurotoxicity evaluation model, a chemical hepatotoxicity evaluation model, a chemical nephrotoxicity evaluation model, a chemical embryogenic toxicity evaluation model, and an angiogenesis model or a cancer infiltration model for drug development.
<9> A tissue model producing method comprising the step of seeding one or more types of cells on the dried vitrigel membrane inside the single cell-culture chamber, and culturing the cells in a single layer or multiple layers.
<10> A tissue model constructed from a single- or multi-layer culture of cells seeded on both surfaces of the dried vitrigel membrane inside the double cell-culture chamber.
<11> The tissue model of <10>, wherein the tissue model is any one of a chemical transdermal absorption model, a chemical corneal permeation model, a chemical gastrointestinal absorption model such as in intestinal tract, a chemical airway absorption model such as in lungs, a chemical vascular permeation model, a chemical hepatic metabolism model, a chemical renal glomerular filtration and excretion model, a chemical dermotoxicity evaluation model, a chemical keratotoxicity evaluation model, a chemical oral mucosal toxicity evaluation model, a chemical neurotoxicity evaluation model, a chemical hepatotoxicity evaluation model, a chemical nephrotoxicity evaluation model, a chemical embryogenic toxicity evaluation model, and an angiogenesis model or cancer infiltration model for drug development.
<12> A method for producing a tissue model inside the double cell-culture chamber, the method comprising the steps of:
   seeding cells on the vitrigel membrane from a first chamber side and culturing the cells in a single layer or multiple layers; and
   inverting the culture chamber, and seeding cells on the vitrigel membrane from a second chamber side and culturing the cells in a single layer or multiple layers.
<13> A single cell-culture chamber producing method comprising the steps of:
   (1) covering a substrate with a film detachably provided for a dried vitrigel membrane and forming a hydrogel inside a wall surface mold placed on the film, said wall surface mold being a tubular frame with no top and bottom surfaces, and allowing a part of the free water inside the hydrogel to flow out through a gap between the substrate and the wall surface mold;
   (2) removing the wall surface mold from the substrate;
   (3) drying the hydrogel to remove the remaining free water and produce a vitrified dry hydrogel;
   (4) rehydrating the dry hydrogel to produce a vitrigel membrane;
   (5) redrying the vitrigel membrane to remove free water and produce a vitrified dried vitrigel membrane;
   (6) detaching the dried vitrigel membrane adsorbed to the film from the substrate together with the film, and adhesively securing the dried vitrigel membrane side to one open end surface of a tubular frame with a urethane-based adhesive or with double-sided tape or by heat welding;
   (7) shaping the dried vitrigel membrane in substantially the same shape as the open end surface of the frame; and
   (8) removing the film from the dried vitrigel membrane.
<14> A single cell-culture chamber producing method comprising the steps of:
   (1) forming a support-containing hydrogel in a container, and drying the hydrogel to remove free water and produce a vitrified support-attached dried hydrogel;
   (2) rehydrating the support-attached dry hydrogel to produce a support-attached vitrigel membrane;
   (3) redrying the support-attached vitrigel membrane to remove free water and produce a vitrified support-attached dried vitrigel membrane in the container;
   (4) rehydrating and detaching the support-attached dried vitrigel membrane from the container, and drying the detached membrane held between magnets to produce a support-attached dried vitrigel membrane detached from the substrate;
   (5) adhesively securing the support-attached dried vitrigel membrane to one open end surface of a tubular frame with a urethane-based adhesive or with double-sided tape or by heat welding after being detached from the substrate; and
   (6) shaping the support-attached dried vitrigel membrane in substantially the same shape as the open end surface of the frame.
<15> A single cell-culture chamber producing method comprising the steps of:
   (1) forming a support-containing hydrogel inside a wall surface mold placed on a substrate, and allowing a part of the free water inside the hydrogel to flow out through a gap between the substrate and the wall surface mold;
   (2) removing the wall surface mold from the substrate;
   (3) drying the support-containing hydrogel to remove the remaining free water and produce a vitrified support-attached dry hydrogel;
   (4) rehydrating the support-attached dry hydrogel to produce a support-attached vitrigel membrane;
   (5) redrying the support-attached vitrigel membrane to remove free water and produce a vitrified support-attached dried vitrigel membrane on the substrate;
   (6) rehydrating and detaching the support-attached dried vitrigel membrane from the substrate, and drying the membrane held between magnets to produce a support-attached dried vitrigel membrane detached from the substrate;
   (7) adhesively securing the support-attached dried vitrigel membrane to one open end surface of a tubular frame with a urethane-based adhesive or with double-sided tape or by heat welding after being detached from the substrate; and
   (8) shaping the support-attached dried vitrigel membrane in substantially the same shape as the open end surface of the frame.
<16> A single cell-culture chamber producing method comprising the steps of:
   (1) forming a support-containing hydrogel inside a container, and drying the hydrogel to remove free water and produce a vitrified support-attached dry hydrogel;
   (2) rehydrating the support-attached dry hydrogel to produce a support-attached vitrigel membrane;
   (3) redrying the support-attached vitrigel membrane to remove free water and produce a vitrified support-attached dried vitrigel membrane in the container;
   (4) rehydrating and detaching the support-attached dried vitrigel membrane from the container, and mounting the detached membrane on a film;
   (5) adhesively securing the rehydrated support-attached vitrigel membrane layered on the film to one open end surface of a tubular frame with a urethane-based adhesive or with double-sided tape or by heat welding;
   (6) drying the layered support-attached vitrigel membrane on the film to produce a support-attached dried vitrigel membrane layered on the film;
   (7) shaping the layered support-attached dried vitrigel membrane on the film in substantially the same shape as the open end surface of the frame; and
   (8) removing the film from the support-attached dried vitrigel membrane.
<17> A double cell-culture chamber producing method comprising the step of contacting the tubular frame of the single cell-culture chamber produced by using the method of any one of <13> to <16> to an open end surface of another tubular frame of the same planar cross-sectional shape as that of the tubular frame from the surface side not adhering to the adhesively secured dried vitrigel membrane or support-attached dried vitrigel membrane, and joining and securing the two tubular frames to each other with the dried vitrigel membrane or the support-attached dried vitrigel membrane interposed therebetween.

### Advantage of the Invention

The cell culture chamber of the present invention can be used to easily construct various tissue models reflective of biological tissues and organ units by utilizing the vitrigel properties (including permeability of macromolecular substance, sustained-release of physiologically active substances such as protein, transparency, fiber density similar to those found in the body, and stability).

For example, by taking the epithelium-mesenchyme-endothelium as the minimum unit of each organ, the chemical behavior can be classified into a pathway from the epithelium side to the mesenchyme-endothelium side as in the skin and cornea, and a pathway from the endothelium side to the mesenchyme-epithelium side as in the case of a drug administered into the blood vessel.

The tissue model of the present invention is constructed as, for example, a "tissue sheet (one-cell)" model configured solely from epithelium cells or endothelium cell first exposed to chemicals, an "organoid plate (two-cell)" model configured from two cell types--epithelium cell and mesenchymal cell, or endothelium cell and mesenchymal cell--including mesenchymal cells exposed to chemicals after the epithelium cells or endothelium cells, or an " organoid plate (three-cell)" model configured from three cell types--epithelium cell, mesenchymal cell, and endothelium cell--exposed to chemicals along with the passage of the chemicals.

The tissue model of the present invention constructed in a chamber by using a vitrigel membrane as a culture support can thus be used to pharmacologically, biochemically, molecular biologically, or tissue pathologically analyze the ADMET behaviors by reflecting the passage ways of chemicals, without using laboratory animals. Further, the tissue model of the present invention can also be used for the biochemical, molecular biological, or tissue pathological analysis of not only the interactions between cells but the effects of various exogenous physiologically active substances.

### Brief Description of Drawings

FIG. 1 is a flowchart representing an exemplary embodiment of a dried vitrigel membrane producing method.
FIG. 2 is a perspective view representing an example of a wall surface mold used for the dried vitrigel membrane producing method.
FIG. 3(A) is a perspective view representing an exemplary embodiment of a single cell-culture chamber producing method of the present invention, and FIG. 3(B) is a photograph representing the exemplary embodiment of the single cell-culture chamber producing method of the present invention.
FIG. 4(A) is a perspective view representing an exemplary embodiment of a single cell-culture chamber of the present invention, and FIG. 4(B) is a photograph representing the same.
FIG. 5(A) is a schematic cross sectional view representing an example of the single cell-culture chamber of the present invention inserted in a container, and FIG. 5(B) is a photograph representing the same.
FIG. 6 is a schematic cross sectional view representing an example of a tissue model produced with the single cell-culture chamber of the present invention.
FIG. 7 is a schematic cross sectional view representing an example of a tissue model produced with the single cell-culture chamber of the present invention.
FIG. 8 is a schematic cross sectional view representing an example of cells cultured in the "liquid phase-vitrigel membrane-gas phase" state using the single cell-culture chamber of the present invention.
FIG. 9(A) is a perspective view representing an exemplary embodiment of a double cell-culture chamber producing method of the present invention, and FIG. 9(B) is a perspective view representing an exemplary embodiment of the double cell-culture chamber of the present invention.
FIG. 10 is a photograph representing an exemplary embodiment of the double cell-culture chamber of the present invention.
FIG. 11 is a schematic cross sectional view representing an example of a tissue model produced with the double cell-culture chamber of the present invention.
FIG. 12 is a schematic cross sectional view representing an example of a vitrigel chamber hung and held in a container for the evaluation of protein permeability.
FIG. 13 is a schematic cross sectional view representing an example of PC-12 cells held in a vitrigel chamber and acted upon by NGF via the vitrigel membrane for the evaluation of protein permeability through the vitrigel chamber.
FIG. 14 represents the neurite extension of the PC-12 cells acted upon by NGF through the vitrigel membrane (upper column), and the state of PC-12 cells used with a commercially available collagen membrane chamber (lower column).
FIG. 15 is a schematic view representing the steps of constructing a tissue model using the single cell-culture chamber.
FIG. 16 is a diagram representing stained images of frozen sections of a cultured cornea model.
FIG. 17 is a diagram representing the result of the evaluation of eye irritant substances using a human corneal epithelium model.
FIG. 18 represents photographs showing cells in each layer corresponding to the example schematically represented in FIG. 11.
FIG. 19 represents a frozen section of a corneal epithelium model produced with a commercially available PET membrane chamber as observed under a phase-contrast microscope, showing that the slice is divided at the PET membrane portion, and that the PET membrane and the cell layer are detached.
FIG. 20 schematically represents a cross section of a tissue model (organoid plate constructed from two sheets of collagen vitrigel membrane and three cell types) produced by using the double cell-culture chamber, along with a stained image of a frozen section of the tissue model.
FIG. 21 is a flowchart representing an exemplary embodiment of a conventional thin vitrigel membrane producing method.

### Description of Embodiments

First Embodiment of the single cell-culture chamber producing method of the present invention is described below.

The dried vitrigel membrane used for the single cell-culture chamber of the present invention can be produced, for example, through the following steps (1) to (5) of:
(1) covering a substrate with a film detachably provided for a dried vitrigel membrane and forming a hydrogel inside a wall surface mold placeed on the film, and allowing a part of the free water inside the hydrogel to flow out through a gap between the substrate and the wall surface mold;
(2) removing the wall surface mold from the substrate;
(3) drying the hydrogel to remove the remaining free water and produce a vitrified dry hydrogel;
(4) rehydrating the dry hydrogel to produce a vitrigel membrane; and
(5) redrying the vitrigel membrane to remove free water and produce a vitrified dried vitrigel membrane.

As used herein, "hydrogel" refers to a substance of a mesh structure formed by the chemical bonding of polymers and holding large amounts of water in the mesh. More specifically, "hydrogel" is a gel obtained after introducing crosslinkage in naturally derived polymers, synthetic polymers, or other artificial materials.

By "dry hydrogel", it means a hydrogel vitrified by removing free water. The term "vitrigel membrane" refers to a rehydrated membrane of the dry hydrogel. As mentioned above, the novel stable-state gel that can be produced through a vitrification step has been named a "vitrigel" by the present inventors. The "dried vitrigel membrane" is a membrane obtained after revitrification of the vitrigel. The dried vitrigel membrane can be converted into the vitrigel membrane by being rehydrated, as needed.

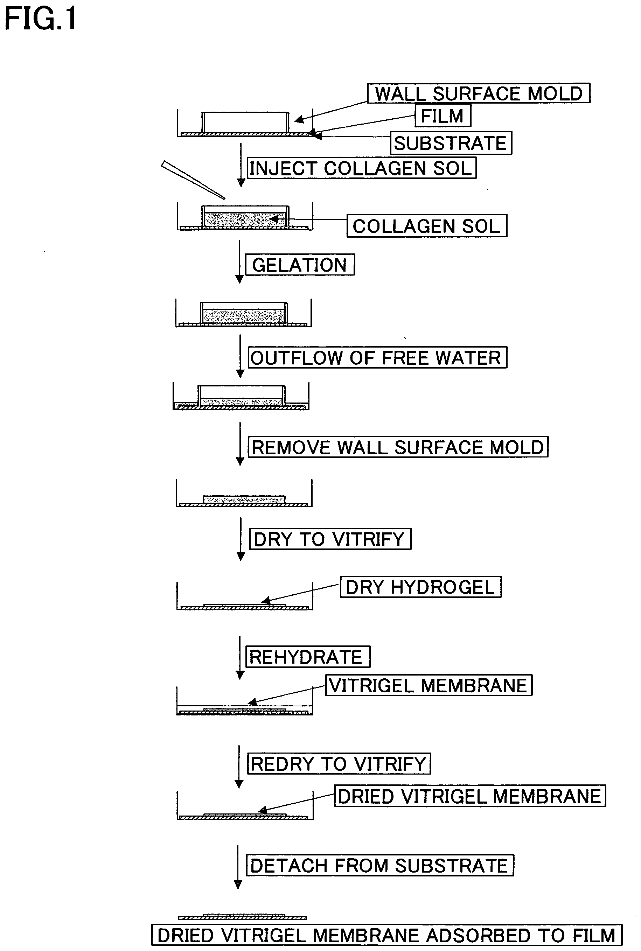

Each step is described below. FIG. 1 is a flowchart representing an exemplary embodiment of the dried vitrigel membrane producing method. In the example of FIG. 1, the sol is described as being a collagen sol.

Step (1): The substrate is covered with a film detachably provided for a dried vitrigel membrane, and a wall surface mold is placed on the film. Then, a sol is injected into the wall surface mold. After the gelation, a part of the free water inside the hydrogel is flown through gaps between the substrate and the wall surface mold.

Materials that can withstand sterilization with, for example, 70% ethanol or in an autoclave can be appropriately used for the substrate and the wall surface mold. Specific examples of such materials include plastics such as polystyrene and acryl, and glass and stainless steel.

Examples of the film detachably provided for the dried vitrigel membrane include non-water-absorbing films such as a parafilm, polyethylene, polypropylene, Teflon^{®}, silicon, Saran Wrap, and vinyl. Particularly preferred is a parafilm. The parafilm is a thermoplastic film using paraffin as the raw material, and, with its elasticity and tackiness, provides excellent airtightness and waterproof performance. In the following, the term "film" will be used to refer to all such films.

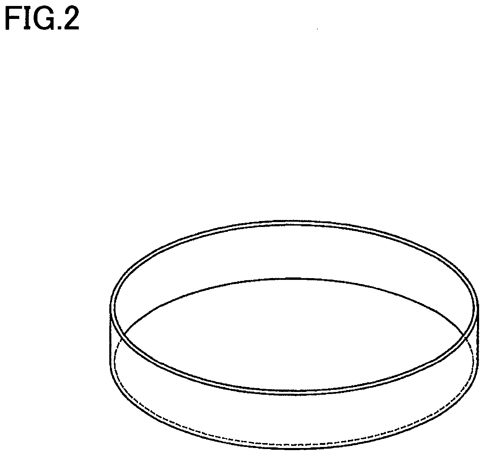

The wall surface mold may be provided as, for example, a tubular frame with no top and bottom surfaces, and may have the same shape as the shape of the desired vitrigel membrane. Specifically, for example, when a circular vitrigel membrane is to be produced, a mold with a cyclic (cylindrical) wall (frame) may be used, as illustrated in FIG. 2. When producing a rectangular vitrigel membrane, the mold may have a rectangular (rectangular tube) wall (frame).

The wall surface mold is placed on the film covering the substrate. Here, the region covered by the film is larger than the cross section of the wall surface mold, and the film is in contact with the bottom surface of the wall surface mold. Physically, however, small gaps large enough to cause an outflow of the free water are formed because of the surface irregularities of the film and the wall surface mold. More than one wall surface molds may be placed on the film as may be decided according to the desired number of vitrigel membranes.

Examples of the naturally derived polymers used as the raw material of the hydrogel production include collagens (including collagen I, II, III, V, and XI), a basement membrane component (product name: Matrigel) reconstructed from mouse EHS tumor extracts (including collagen IV, laminin, and heparan sulfate proteoglycan), gelatin, agar, agarose, fibrin, glycosaminoglycan, hyaluronan, and proteoglycan . An optimum component (such as a salt) for the gelation of each material, the concentration of such components, and pH may be selected for the hydrogel production. Vitrigel membranes that mimic various types of biological tissues can be obtained by combining different raw materials.

Examples of the synthetic polymer used for the hydrogel production include polyacrylamide, polyvinyl alcohol, methyl cellulose, polyethyleneoxide, and poly(ll-hydroxyethylmethacrylate)/polycaprolactone. Two or more of these polymers may be used to produce the hydrogel. The hydrogel amount may be adjusted taking into account the thickness of the product vitrigel membrane.

Collagen is particularly preferable as the raw material of the hydrogel. When using a collagen gel, a collagen sol may be injected into the wall surface mold placed on the substrate, and formed into a gel in an incubator. In the example of FIG. 1, the collagen sol is used as the hydrogel raw material.

Taking the collagen sol as an example, the collagen sol may be prepared in solutions having optimum salt concentrations, including, for example, physiological saline, PBS (Phosphate Buffered Saline), HBSS (Hank's Balanced Salt Solution), basal culture medium, serum-free culture medium, serum-containing culture medium. The pH of the solution for forming the collagen gel is preferably about 6 to about 8.

Desirably, the collagen sol is prepared at 4°C. The maintained temperature for the gelation needs to be lower than the collagen denaturation temperature, which depends on the animal species from which the collagen was obtained. Generally, the collagen sol may be maintained at a temperature of 37°C or less to allow the gelation to complete in several minutes to several ten minutes.

The collagen sol gelates only weakly at an excessively low collagen concentration of 0.2% or less. A collagen concentration of 0.3% or more is too high to ensure uniformly. The collagen concentration of the collagen sol is preferably 0.2 to 0.3%, more preferably about 0.25%.

The collagen sol adjusted as above is injected into the wall surface mold. Because the collagen sol of the foregoing concentrations is viscous, the collagen sol can gelate within several minutes without flowing out through the gaps between the substrate and the wall surface mold if heated quickly after being injected into the wall surface mold.

The resulting collagen gel adheres to the substrate and the wall surface mold. When left unattended for a predetermined time period, some of the free water inside the collagen gel flows out of the wall surface mold over time through the gaps between the substrate and the wall surface mold. Here, the outflow of the free water can be promoted by slightly moving the wall surface mold (for example, up and down), because it releases the adhesion between the gel and the wall surface mold and creates a small gap.

Further, for example, when the amount of the 0.25% collagen sol injected per unit area (1.0 cm²) is 0.4 ml or more, it is desirable to remove the free water over a time course as it flows out through the gaps between the substrate and the wall surface mold, until the free water inside the collagen gel is reduced to about 1/4 to about 2/3. This makes the gel collagen concentration about 0.375 to about 1.0%, providing a gel strength that does not cause distortion in the gel shape even after the removal of the wall surface mold. Subsequently, the free water remaining in the gel can be removed for vitrification by being naturally dried along with the free water that has flown onto the substrate. From the standpoint of quick mass production, the free water inside the collagen gel is reduced to about 1/4 to about 2/3 in desirably 2 to 8 hours. The subsequent removal of the remaining free water in the gel by natural drying desirably completes within 48 hours. To this end, the amount of the 0.25% collagen sol injected per unit area (1.0 cm²) is desirably 0.1 to 2.4 ml. In this way, the product collagen vitrigel membrane can contain 250 µg to 6 mg of collagen per unit area (1.0 cm²).

Step (2): The wall surface mold is removed from the substrate.

The wall surface mold is removed leaving the hydrogel on the film covering the substrate. Because the free water has flown out, the hydrogel does not undergo deformation or other changes on the film, and can maintain the shape given by the wall surface mold.

Step (3): The hydrogel is dried to remove the remaining free water and produce a vitrified dry hydrogel.

The free water inside the hydrogel is completely removed by drying to vitrify the hydrogel. A vitrigel membrane of improved transparency and strength can be obtained upon rehydration by increasing the duration of the vitrifying step. If need be, the vitrigel membrane obtained upon rehydration after a brief period of vitrification may be washed with PBS or the like, and revitrified.

Various drying methods can be used, including, for example, air drying, drying in a sealed container (air inside a container is circulated to provide a constant supply of dry air), and drying in an environment with a silica gel. As an example of air drying, the hydrogel may be dried for 2 days in a germ-free incubator at 10°C and 40% humidity, or may be dried for a whole day in a germ-free clean bench at room temperature.

Step (4): The dry hydrogel is rehydrated to produce a vitrigel membrane.

The vitrigel membrane can be produced by rehydrating the dry hydrogel with PBS or the culture medium used. Here, the liquid used for rehydration may contain various components such as a physiologically active substance. Examples of such physiologically active substances include antibiotics and various pharmaceutical preparations, cell growth factors, differentiation inducing factors, cell adhesion factors, antibodies, enzymes, cytokines, hormones, and lectins. Extracellular matrix components that do not under gelation, for example, such as fibronectin, vitronectin, entactin, and osteopontin also may be contained. More than one of these components may be contained.

Step (5): The vitrigel membrane is redried to remove free water and produce a vitrified dried vitrigel membrane.

As in step (3), various drying methods can be used, including, for example, air drying, drying in a sealed container (air inside a container is circulated to provide a constant supply of dry air), and drying in an environment with a silica gel.

The vitrified dried vitrigel membrane can be produced upon redrying the vitrigel membrane. The dried vitrigel membrane can be reconverted into the vitrigel membrane by being rehydrated, as needed.

Because the dried vitrigel membrane is laminated with the detachable film, the dried vitrigel membrane can be freely handled with the film, and the vitrified dried vitrigel membrane can be cut into any shape.

It should be noted that the components contained in the "dry hydrogel" and the "dried vitrigel membrane" are not necessarily the same. The "dry hydrogel" contains the hydrogel components, whereas the "dried vitrigel membrane" contains the components remaining in the vitrigel membrane equilibrated with the aqueous solution used for the rehydration of the dry hydrogel.

The vitrigel membrane obtained by rehydrating the dried vitrigel membrane in step (5) undergoes a longer vitrification period than the vitrigel membrane obtained in step (4), and thus has superior strength and transparency.

The duration of the vitrification period may extend in the "dry hydrogel" state. However, the "dry hydrogel" state contains all the components that are present in the hydrogel production, including components unnecessary for maintaining the dry hydrogel or using the vitrigel membrane. On the other hand, the vitrigel membrane after the removal of the unnecessary components by the rehydration of the "dry hydrogel" does not contain unnecessary components for the dry product. It is therefore preferable to maintain the vitrification period in the dried vitrigel membrane state if the vitrification period is to be maintained for extended time periods. The vitrigel membrane obtained by the rehydration of the dried vitrigel membrane is thus desirable because of the absence of the unnecessary components.

The dried vitrigel membrane can also be produced, for example, by mixing the desired physiologically active substance with the sol solution before gelation, followed by the vitrigel membrane producing step that involves gelation, vitrification, and other procedures.

The dried vitrigel membrane containing a physiologically active substance can realize a more desirable culture environment, because it allows the factors necessary for, for example, cell adhesion, proliferation, and differentiation to be supplied from the vitrigel membrane side. The dried vitrigel membrane containing a physiologically active substance is also highly useful for the testing conducted to examine the effects of the contained physiologically active substance on cells. Further, the vitrigel membrane containing a physiologically active substance can function as a drug delivery system upon being transplanted in the body (NPL 2).

Further, the vitrigel membrane allows for passage of physiologically active substances of large molecular weights. This greatly contributes to the testing and studies of physiologically active substance-mediated interactions between cells seeded on two different surfaces of the vitrigel membrane (NPL 2).

With the foregoing method, the dried vitrigel membrane can be obtained in the membrane state, without adhering to the culture Petri dish. Further, because the dried vitrigel membrane can easily be cut, it can be used for the cell culture chamber of the present invention.

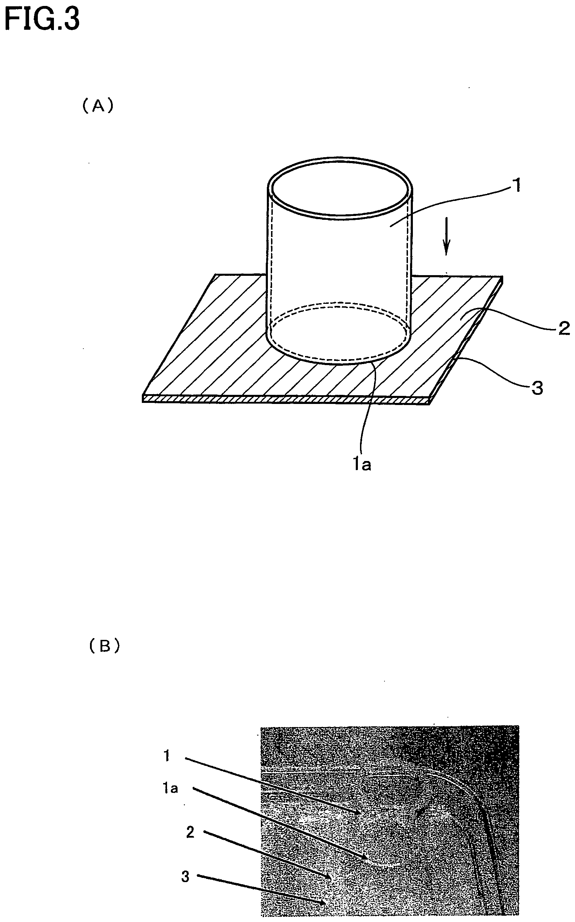

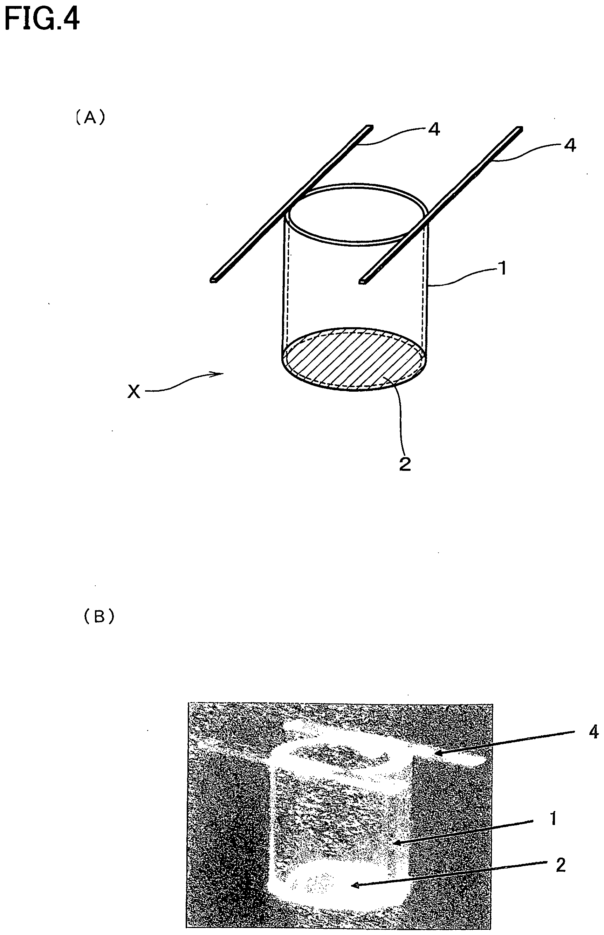

FIG. 3(A) is a perspective view representing an embodiment of the single cell-culture chamber producing method of the present invention. FIG. 3(B) is a photograph representing the embodiment of the single cell-culture chamber producing method of the present invention. FIG. 4(A) is a perspective view representing an embodiment of the single cell-culture chamber of the present invention. FIG. 4(B) is a photograph representing the embodiment of the single cell-culture chamber of the present invention.

A single cell-culture chamber X has a tubular frame 1, and a dried vitrigel membrane 2 covering and being secured on one open end surface 1a of the tubular frame 1 (FIG. 4, (A) and (B)).

As illustrated in FIG. 3, (A) and (B), the tubular frame 1 has a space formed therein for holding cells. Materials suited for cell culture can be appropriately selected for the material of the frame 1. The shape of the frame 1 is not particularly limited either, and may be, for example, cylindrical or a rectangular tubular shape. Specifically, preferred as the frame 1 is, for example, an acrylic or polystyrene cylindrical tube.

For example, an adhesive is applied to the open end surface 1a of the frame 1, and the dried vitrigel membrane 2 is adhesively secured. The shape of the open end surface is not particularly limited, and may be, for example, flat, stepped, tapered, or grooved. When the dried vitrigel membrane 2 is used as a laminate with a film 3, the film 3 can be peeled and removed after bonding the dried vitrigel membrane 2 side to the frame 1. The adhesive may be appropriately selected taking into consideration bondability and cytotoxicity. Specifically, for example, urethane-based adhesives are used. For example, rubber, cyanacrylate, and acrylic adhesives are not preferable, because these may exhibit cytotoxicity. Hot melt adhesives are not preferred either, because it may heat denature the dried vitrigel membrane 2. The dried vitrigel membrane 2 and the frame 1 may be adhesively secured by using various methods, including, for example, a method that adhesively secures the dried vitrigel membrane 2 and the frame 1 with a double-sided tape interposed therebetween, and a method that heat fuses the dried vitrigel membrane 2 and the frame 1 by using, for example, a heat sealer, a hot plate, ultrasonic waves, and a laser.

The dried vitrigel membrane 2 adhesively secured to the end surface 1a of the frame 1 can be cut into substantially the same shape as the shape of the end surface 1a of the frame 1. When provided as a laminate with the film 3, the dried vitrigel membrane 2 can easily be cut, and the unnecessary portions protruding from the open end surface 1a of the frame 1 can be cut off. The film 3 can be peeled and removed as above after cutting the dried vitrigel membrane 2. In this way, it is possible to produce a single cell-culture chamber that has excellent ease of handling, as illustrated in FIG. 4, (A) and (B).

A tissue model can be constructed by seeding and culturing the desired cells on the dried vitrigel membrane 2 inside the single cell-culture chamber X. The dried vitrigel membrane 2 inside the single cell-culture chamber X is converted into the vitrigel membrane upon adding a cell-containing suspension or culture medium. As used herein, "tissue model" refers to models mimicking the cell state, tissues, and organs of the body, and can be used, for example, for assaying the effects of physiologically active substances (including drugs such as various pharmaceutical preparations, and nutrition components and growth factors) on tissues (cells).

The tissue model can be constructed, for example, by seeding and culturing various mammal-derived cells, preferably, human-derived cells. A tissue model using human-derived cells can be used to establish an evaluation system that can overcome the problem of species difference in examining the ADMET (absorptiondistribution-metabolismexcretiontoxicity) of chemicals against humans.

The form of the tissue model is not limited, and may be, for example, an epithelial tissue model that can be constructed by culturing, for example, surface epithelial cells and glandular epithelial cells; a connective tissue model that can be constructed by culturing, for example, fibroblasts and fat cells; a muscle tissue model that can be constructed by culturing, for example, myoblasts, cardiac muscle cells, and smooth muscle cells; a nerve tissue model that can be constructed by culturing, for example, nerve cells and glial cells; and an organoid model that can be constructed from a combination of cells derived from two or more tissues. The cells used are not limited to normal mature differentiated cells, and may be undifferentiated cells such as embryonic stem (ES) cells, somatic stem cells, and induced pluripotent stem (iPS) cells; focus-derived cells such as cancer cells; or transformants transfected by exogeneous genes. By appropriately selecting cells, it is possible to create not only a normal tissue model but other forms of tissue model such as a tissue model of development or reproduction processes, a tissue model of cancers and other lesions, and a tissue model configured from artificially transformed cells. In a form of tissue model with which the effects of chemicals such as pharmaceutical preparations, physiologically active substances, cosmetics, and detergents on the body can be extrapolated, it is important to construct a tissue model that can reflect the passage way of the chemical exposed or administered to the body. From this perspective, the following tissue models can be exemplified:
A "tissue sheet (one-cell)" model configured solely from epithelium cells or endothelium cell first exposed to chemicals.

An "organoid plate (two-cell)" model configured from two cell types--epithelium cell and mesenchymal cell, or endothelium cell and mesenchymal cell--including mesenchymal cells exposed to chemicals after the epithelium cells or endothelium cells.

An "organoid plate (three-cell)" model configured from three cell types--epithelium cell, mesenchymal cell, and endothelium cell--exposed to chemicals along with the passage of the chemicals.

Specific examples of the tissue model include mature tissue models for various organs (such as skin, cornea, oral mucosa, nerve, liver, and kidneys) useful for the toxicity evaluation of chemicals, including a chemical transdermal absorption model, a chemical corneal permeation model, a chemical gastrointestinal absorption model such as in intestinal tract, a chemical airway absorption model such as in lungs, a chemical vascular permeation model, a chemical hepatic metabolism model, and a chemical renal glomerular filtration and excretion model. Other examples include embryonic tissue models useful for the developmental toxicity evaluation of chemicals, and angiogenesis models and cancer infiltration models useful for drug development.

The assay method used for the tissue model is not limited to a specific method, and may be, for example, a method that directly adds chemicals into the chamber, or a method that allows chemicals to act on the cells by taking advantage of the permeability of the vitrigel membrane.

As an example of the method that takes advantage of the permeability of the vitrigel membrane, a method may be used that assays the effects of a physiologically active substance on cells by permeating the cultured cells with the physiologically active substance via the vitrigel membrane inside a container after injecting the physiologically active substance into the container and placing the single cell-culture chamber therein.

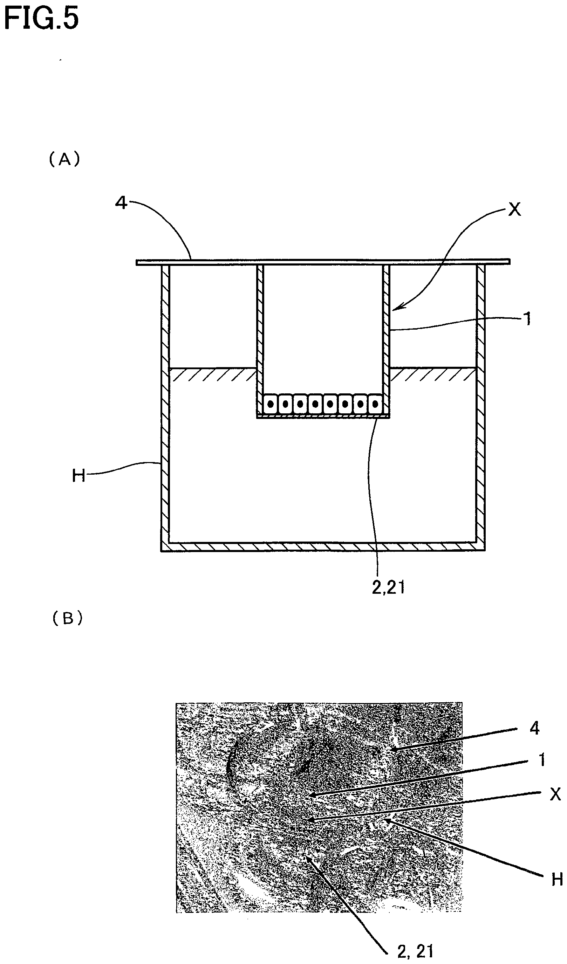

Specifically, for example, as illustrated in FIG. 5, outwardly protruding stoppers 4 are provided on the outer periphery portions of the open end surface of the frame 1 opposite the end surface covered with and secured to the dried vitrigel membrane 2 (FIG. 4, (A) and (B)). The single cell-culture chamber X is then inserted into the container H from the above. The single cell-culture chamber X can be held inside the container H by being hung on the container H with the stoppers 4 provided on the frame 1. The stoppers 4 are not limited to the form shown in FIG. 1, and may be, for example, a rod-like, or a flange-like member made of material such as plastic material. The physiologically active substance may be appropriately injected into the container H to assay the effects of the physiologically active substance on the cultured cells permeated with the physiologically active substance via the vitrigel membrane.

When using a dried vitrigel membrane that contains the physiologically active substance in advance, the physiologically active substance can be supplied to the culture cells inside the chamber via the hydrated vitrigel membrane upon culturing the desired cells on the dried vitrigel membrane, and the effects of the physiologically active substance can be assayed.

The vitrigel membrane is softer than plastic films such as PET, and allows frozen sections of the tissue model to be easily produced. This makes it possible to three-dimensionally observe the effects of the physiologically active substance on the tissue model.

Various tissue models may be constructed in the single cell-culture chamber X for different purposes.

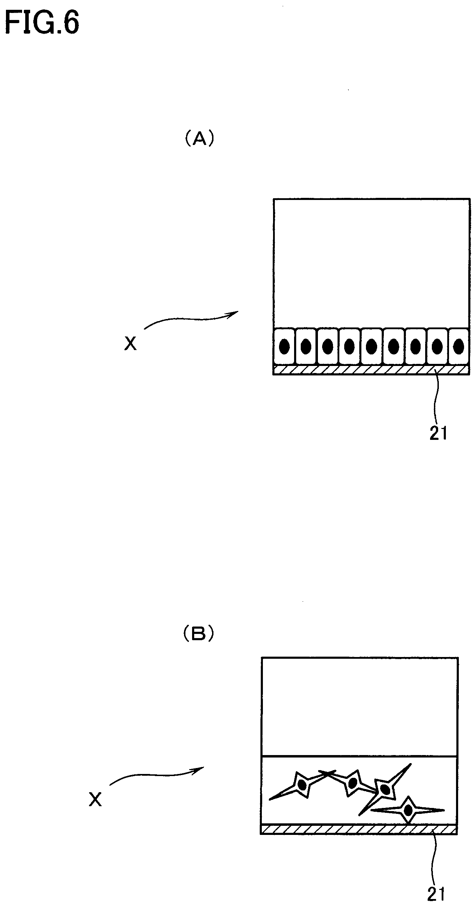

Specifically, for example, as shown in FIG. 6(A), more than one types of desired cells may be seeded on a vitrigel membrane 21 in the single cell-culture chamber X. A tissue model can be constructed on the vitrigel membrane 21 inside the single cell-culture chamber X by culturing the seeded cells in a single layer or multiple layers under preferred conditions.

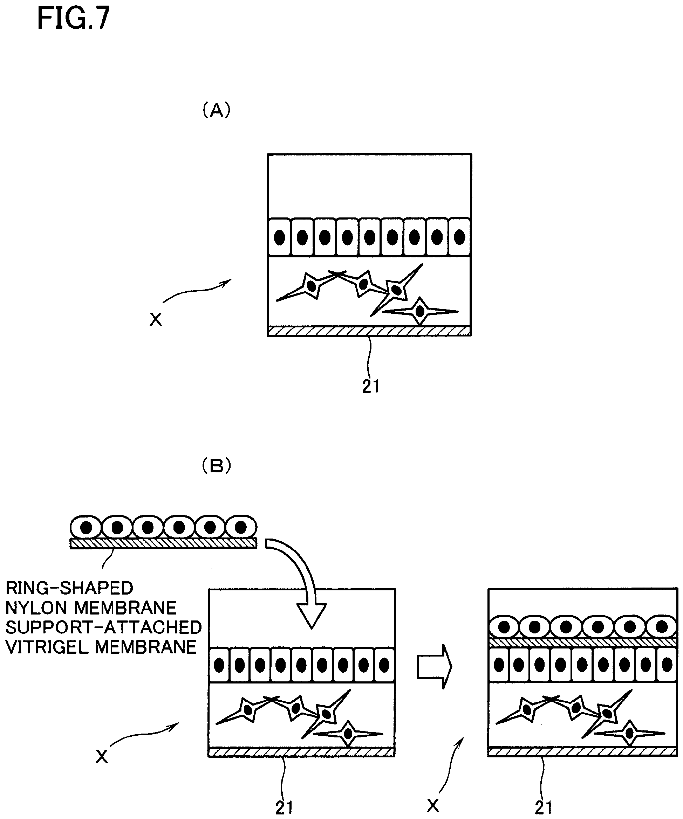

Further, for example, as shown in FIG. 6(B), a collagen culture medium (collagen sol) suspending one or more types of cells may be added onto the vitrigel membrane 21 in the single cell-culture chamber X, and the cells may be cultured to construct a tissue model culturing the cells in the collagen gel on the vitrigel membrane 21. Further, a culture medium suspending different cells may be added onto the tissue model shown in FIG. 6(B), and the cells may be grown in overlay culture in a single layer or multiple layers to construct a tissue model as shown in, for example, FIG. 7(A).

Further, for example, one or more types of cells may be cultured on one side or both sides of a vitrigel membrane embedded with a ring-shaped nylon membrane support produced by using a conventional method, and the vitrigel membrane may be subjected to overlay culture on the tissue model shown in FIG. 6(A) or (B), or in FIG. 7(A) to construct a multilayer tissue model. Note that FIG. 7(B) represents the state of the overlay culture on the tissue model shown in FIG. 7(A).

As described above, the single cell-culture chamber of the present invention can be used to easily construct various three-dimensional tissue models reflective of biological tissues and organ units by utilizing the vitrigel properties (including permeability of macromolecular substance, sustained-release of physiologically active substances such as protein, transparency, fiber density similar to those found in the body, and stability). Specifically, it is possible to easily construct, for example, a "tissue sheet (one-cell)" model configured solely from epithelium cells or endothelium cell first exposed to chemicals, an "organoid plate (two-cell)" model configured from two cell types--epithelium cell and mesenchymal cell, or endothelium cell and mesenchymal cell--including mesenchymal cells exposed to chemicals after the epithelium cells or endothelium cells, or an "organoid plate (three-cell)" model configured from three cell types--epithelium cell, mesenchymal cell, and endothelium cell--exposed to chemicals along with the passage of the chemicals.

With the tissue model constructed inside the chamber using the vitrigel membrane as a culture support, it is therefore possible to pharmacologically, biochemically, molecular biologically, or tissue pathologically analyze the ADMET behaviors by reflecting the passage ways of chemicals, without using laboratory animals. Further, with the tissue model constructed inside the chamber using the vitrigel membrane as a culture support, it is possible to biochemically, molecular biologically, or tissue pathologically analyze not only cell-to-cell interactions but the effects of various exogenous physiologically active substances, without using laboratory animals.

Exemplary usages of the single cell-culture chamber are described below.

For example, cells can be cultured in the "liquid phase-vitrigel membrane-liquid phase" state by placing the chamber in a culture medium-containing container after the desired cells suspended in a culture medium are seeded on the vitrigel membrane inside the chamber. Further, cells can be cultured in the "gas phase-vitrigel membrane-liquid phase" state by removing the culture medium inside the chamber. The "gas phase-vitrigel membrane-liquid phase" culture method is suited, for example, for the culturing of the epithelium cells in the skin, mouth, nasal cavity, and lungs that are usually in contact with air in the body, and can culture the cells for extended time periods with the maintained cell functions.

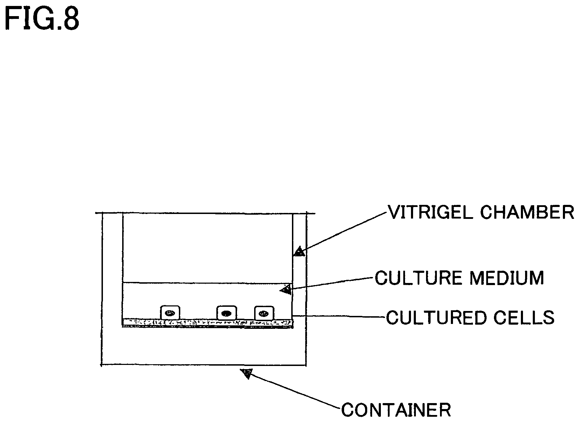

Further, for example, as shown in FIG. 8, the single cell-culture chamber with the culture medium may be held in the air inside an empty container by being hung on the upper portion of the container with the stoppers. In this way, the cells on the vitrigel membrane can contact the culture medium inside the chamber and the ambient air via the vitrigel membrane, and can thus be cultured with the desirable supply of oxygen from the lower side of the cells (the vitrigel membrane side representing the cell adhesion surface) in the "liquid phase-vitrigel membrane-gas phase" state. The "liquid phase-vitrigel membrane-gas phase" culture method is also suited for the culturing of, for example, the oxygen-demanding hepatic parenchymal cells, nerve cells, and cancer cells, and can culture the cells for extended time periods with the maintained cell functions.

Further, cells can be cultured in the "liquid phase-vitrigel membrane-solid phase" state by being cultured on the vitrigel membrane with the outer surface of the vitrigel membrane in the culture medium-containing single cell-culture chamber in contact with the container or other solid materials.

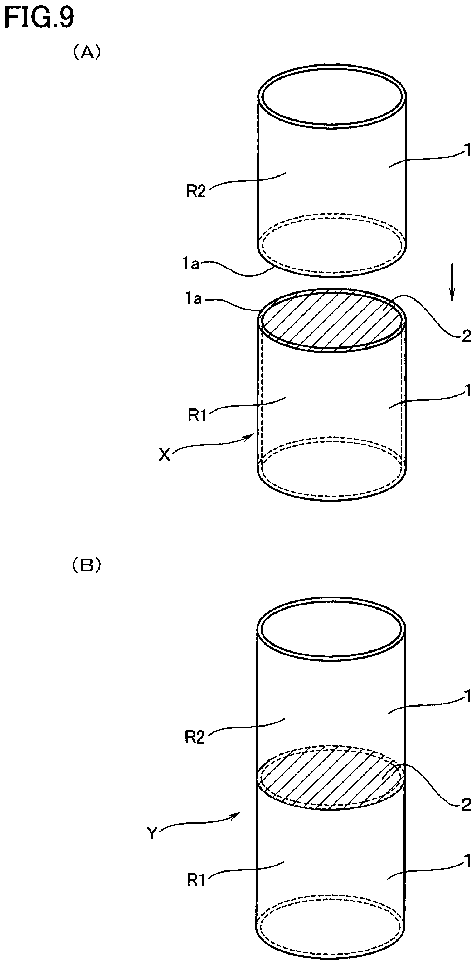

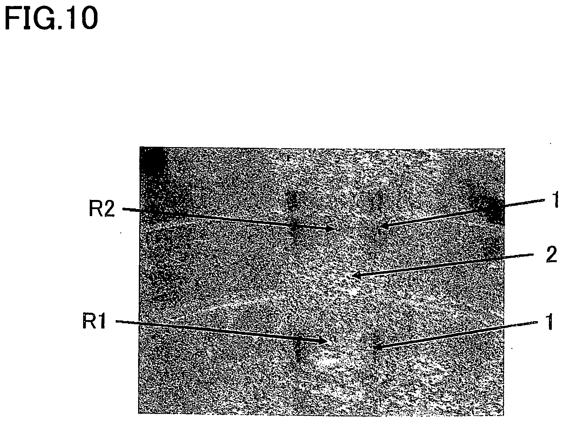

FIG. 9(A) is a perspective view representing an embodiment of the double cell-culture chamber producing method of the present invention. FIG. 9(B) is a perspective view representing an embodiment of the double cell-culture chamber of the present invention. FIG. 10 is a photograph representing the embodiment of the double cell-culture chamber of the present invention.

A double cell-culture chamber Y of the present invention is constructed from two tubular frames 1 adhesively secured to each other with the dried vitrigel membrane 2 interposed between the opposing open end surfaces 1a, and has two chambers parted by the dried vitrigel membrane 2 (hereinafter, also referred to as a first chamber and a second chamber for convenience). The shape of the tubular frames 1 is not particularly limited, as long as water tightness is maintained for the two chambers formed (as long as the liquid is not leaked). For example, the tubular frames 1 may be of different cross sectional shapes, heights, diameters, and thicknesses as may be appropriately selected. Preferably, for example, two frames 1 of the same planar shape may be used.

Specifically, for example, the single cell-culture chamber X is produced by using the foregoing method, and the double cell-culture chamber Y may be produced by forming a first chamber R1 and a second chamber R2 via the dried vitrigel membrane 2 upon bringing the frames 1 of the same shape to contact and adhere to each other on the side of the surface of the dried vitrigel membrane 2 (the outer surface side of the dried vitrigel membrane 2) not adhering to the frame 1 of the single cell-culture chamber X. The two frames 1 may be joined to each other via the dried vitrigel membrane 2 by appropriately using, for example, a urethane-based adhesive or a double-sided tape, as in the case of the production of the single cell-culture chamber X. Further, for example, the frames 1 may be joined by adhesively securing the opposing surfaces from outside by using a film-shaped adhesive material such as a parafilm. It is also possible, for example, to thread the open end surfaces 1a of the frames 1, and fasten and fix the frames 1 with a structure similar to a screw cap or a snap cap.

The adhesion between the frames 1 of the double cell-culture chamber Y may be releasable.

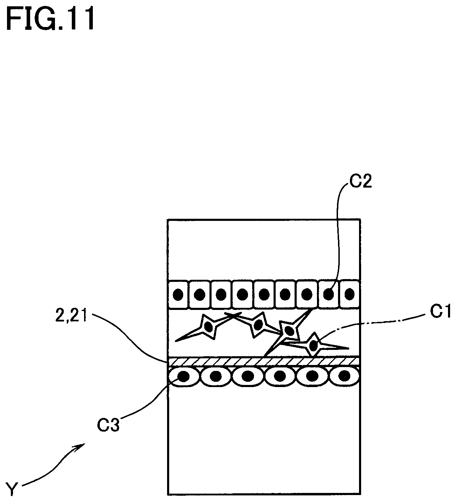

As shown in FIG. 11, one or more types of cells may be cultured in a single layer or multiple layers on one or both surfaces of the vitrigel membrane 21 in the double cell-culture chamber Y. As with the case of the example described with reference to FIG. 7(B) and elsewhere, the culture includes, for example, a gel culture prepared by adding a collagen culture medium suspending one or more cells. In the double-sided culture, different cells may be seeded on the two surfaces to be separately cultured in the first chamber and the second chamber. In each chamber, the cells can be cultured in the same manner as in the single culture chamber. In this case, cell-to-cell interactions via the vitrigel membrane 21 can be studied.

In the example shown in FIG. 11, for example, a three-dimensional tissue model may be constructed in which fibroblasts C1 and endothelium cells C2 dispersed in a collagen gel are cultured on one surface of the vitrigel membrane 21, and epithelium cells C3 are cultured on the other surface of the vitrigel membrane 21.

A multilayer tissue model can easily be constructed by seeding and culturing the desired cells on the both surfaces of the vitrigel membrane 21 in the double cell-culture chamber Y. As in the single cell-culture chamber, examples of the tissue model include various organ models useful for the toxicity evaluation of chemicals, including a chemical transdermal absorption model, a chemical corneal permeation model, a chemical intestinal absorption model, a chemical vascular permeation model, a chemical hepatic metabolism model, and a chemical renal glomerular filtration and excretion model. Other examples include angiogenesis models and cancer infiltration models useful for drug development.

Specifically, for example, a transdermal absorption model or an intestinal absorption model of epithelium-mesenchyme interactions can be constructed by culturing cells of the epithelium lineage on one surface of the vitrigel membrane 21 (first chamber side), and mesenchymal cells on the other side (second chamber side). Further, an angiogenesis model or a cancer infiltration model can be constructed and various cell functions can be assayed by culturing vascular endothelial cells on one surface (first chamber side), and cancer cells on the other side (second chamber side). Further, for example, because the collagen vitrigel membrane has a collagen fiber density similar to those found in the body, it is possible to reproduce the properties of the mesenchymes found in the body. Thus, a double cell-culture chamber Y using a vitrigel membrane having substantially the same thickness (500 µm) as the corneal stroma can be used to construct a cornea model containing epithelium, stroma, and endothelium, by forming corneal epithelium cells on one side (first chamber side) and a corneal endothelium cell layer on the other side (second chamber side).

In a culture using the double cell-culture chamber Y, for example, cells may be cultured in a single layer or multiple layers after being seeded on the dried vitrigel membrane 2 from the first chamber side, and then from the second chamber side, cells may be seeded on the vitrigel membrane 21 and cultured in a single layer or multiple layers after inverting the double cell-culture chamber Y upside down. In this way, a multilayer tissue model can be constructed that is layered via the vitrigel membrane 21. For example, epithelium cells are cultured in a single layer or multiple layers (or endothelium cells are cultured in a single layer) on the dried vitrigel membrane in the second chamber of the double cell-culture chamber. After inverting the double cell-culture chamber Y upside down, mesenchymal cells suspended in a collagen sol are seeded on the dried vitrigel membrane in the first chamber and cultured in the collagen gel, and endothelium cells are cultured in a single layer (or epithelium cells are cultured in a single layer or multiple layers) on the collagen gel. In this way, the "organoid plate (three-cell)" model can easily be constructed with the three cell types--epithelium cell, mesenchymal cell, and endothelium cell--in which the exposure to a chemical progresses along with the passage of the chemical.

After constructing a tissue model in the double cell-culture chamber Y, the adhesion between the frames 1 may be released, and the tissue model in the chamber Y may be subjected to various assays as in the case of the single cell-culture chamber. Specifically, for example, as in the method described with reference to FIG. 5, (A) and (B), the frame 1 may be held inside a container with the stoppers provided on the outer periphery of the frame 1. The physiologically active substance or the like injected into the container can then be supplied from the hydrated vitrigel membrane to the cultured cell (tissue model) side inside the chamber to assay the effect of the physiologically active substance.

As described above, the double cell-culture chamber of the present invention can be used to easily construct various three-dimensional tissue models reflective of biological tissues and organ units by utilizing the vitrigel properties (including permeability of macromolecular substance, sustained-release of physiologically active substances such as protein, transparency, fiber density similar to those found in the body, and stability). Specifically, it is possible to easily construct, for example, a "tissue sheet (one-cell)" model configured solely from epithelium cells or endothelium cell first exposed to chemicals, an "organoid plate (two-cell)" model configured from two cell types--epithelium cell and mesenchymal cell, or endothelium cell and mesenchymal cell--including mesenchymal cells exposed to chemicals after the epithelium cells or endothelium cells, or an "organoid plate (three-cell)" model configured from three cell types--epithelium cell, mesenchymal cell, and endothelium cell--exposed to chemicals along with the passage of the chemicals.

With the tissue model constructed inside the chamber using the vitrigel membrane as a culture support, it is therefore possible to pharmacologically, biochemically, molecular biologically, or tissue pathologically analyze the ADMET behaviors by reflecting the passage ways of chemicals, without using laboratory animals. Further, with the tissue model constructed inside the chamber using the vitrigel membrane as a culture support, it is possible to biochemically, molecular biologically, or tissue pathologically analyze not only cell-to-cell interactions but the effects of various exogenous physiologically active substances, without using laboratory animals.

Further, a layered tissue model containing more complex cell layers may be constructed by layering a vitrigel membrane from a tissue model on a different tissue model, specifically by separating a cell layer-forming vitrigel membrane in the tissue model (for example, as shown in FIGS. 6, 7, and 11) from the frame with an instrument such as a dissecting surgical knife, and layering the separated vitrigel membrane on another tissue model. Further, the frame of a tissue model (tissue model A) constructed by using the chamber may be nested with another tissue model (tissue model B) constructed by using a smaller chamber (smaller than the frame inner diameter). In this way, the tissue model B can be layered on the tissue model A.

Second Embodiment of the single cell-culture chamber producing method of the present invention is described below. Parts of the embodiment already described in First Embodiment will not be described further to avoid redundancy.

In Second Embodiment, the method includes, for example, the following steps of:
(1) forming a support-containing hydrogel in a container, and drying the hydrogel to remove free water and produce a vitrified support-attached dry hydrogel;
(2) rehydrating the support-attached dry hydrogel to produce a support-attached vitrigel membrane;
(3) redrying the support-attached vitrigel membrane to remove free water and produce a vitrified support-attached dried vitrigel membrane in the container;
(4) rehydrating and detaching the support-attached dried vitrigel membrane from the container, and drying the detached membrane held between magnets to produce a support-attached dried vitrigel membrane detached from the substrate;
(5) adhesively securing the support-attached dried vitrigel membrane to one open end surface of a tubular frame with a urethane-based adhesive or with double-sided tape or by heat welding after being detached from the substrate; and
(6) shaping the support-attached dried vitrigel membrane in substantially the same shape as the open end surface of the frame.

In Second Embodiment, specifically, for example, a support-attached dry hydrogel containing a cyclic nylon film as a support (hereinafter, "ring-shaped nylon membrane support") is produced in a container such as a Petri dish according to the method of WO2005/014774 filed by the present inventors, and the hydrogel is rehydrated and redried to produce a ring-shaped nylon membrane support-attached dried vitrigel membrane. The procedures including drying and rehydration can be performed by appropriately using the methods described above.

Thereafter, the ring-shaped nylon membrane support-attached dried vitrigel membrane is rehydrated again and detached from the container, and dried between magnets holding the membrane. Preferably, for example, the magnets are ones that can hold the front and back surfaces near the outer periphery of the ring-shaped nylon membrane support-attached dried vitrigel membrane according to the method described in JP-A-2007-185107 (PTL 4) filed by the present inventors. Specifically, the magnets are preferably circular in shape, and may be used after appropriately designing, for example, the size (width) of the outer circle (outer periphery) and the inner circle (inner periphery), taking into consideration the outer diameter or other dimensions of the ring-shaped nylon membrane support-attached dried vitrigel membrane. By being dried between the magnets holding the membrane, the support-attached dried vitrigel membrane detached from the substrate can be obtained without adhering to a container such as a Petri dish. Further, the support-attached dried vitrigel membrane detached from the substrate can be obtained upon removing the film after drying the ring-shaped nylon membrane support-attached vitrigel membrane with the film held between the magnets.

The support-attached dried vitrigel membrane detached from the substrate is then adhesively secured to one open end surface of the tubular frame, and the unnecessary portion protruding from the end surface of the frame is cut to provide substantially the same shape as the end surface. The single cell-culture chamber can be obtained as a result. Note that the support-attached dried vitrigel membrane may be adhesively secured to the tubular frame by appropriately using, for example, an adhesive, a double-sided tape, or a heat sealer, as in First Embodiment.

Third Embodiment of the single cell-culture chamber producing method of the present invention is described below.

In Third Embodiment, the method includes, for example, the following steps of:
(1) forming a support-containing hydrogel inside a wall surface mold placed on a substrate, and allowing a part of the free water inside the hydrogel to flow out through a gap between the substrate and the wall surface mold;
(2) removing the wall surface mold from the substrate;
(3) drying the support-containing hydrogel to remove the remaining free water and produce a vitrified support-attached dry hydrogel;
(4) rehydrating the support-attached dry hydrogel to produce a support-attached vitrigel membrane;
(5) redrying the support-attached vitrigel membrane to remove free water and produce a vitrified support-attached dried vitrigel membrane on the substrate;
(6) rehydrating and detaching the support-attached dried vitrigel membrane from the substrate, and drying the membrane held between magnets to produce a support-attached dried vitrigel membrane detached from the substrate;
(7) adhesively securing the support-attached dried vitrigel membrane to one open end surface of the tubular frame with a urethane-based adhesive or with double-sided tape or by heat welding after being detached from the substrate; and
(8) shaping the support-attached dried vitrigel membrane in substantially the same shape as the open end surface of the frame.

In Third Embodiment, for example, the dried vitrigel membrane with a support such as the cyclic nylon film can be produced by being detached from the substrate, using the wall surface mold shown in FIG. 2. In Third Embodiment, all steps except for using the wall surface mold placed on the substrate can be performed in the same manner as in Second Embodiment.

Fourth Embodiment of the single cell-culture chamber producing method of the present invention is described below.

In Fourth Embodiment, the method includes, for example, the following steps of:
(1) forming a support-containing hydrogel inside a container, and drying the hydrogel to remove free water and produce a vitrified support-attached dry hydrogel;
(2) rehydrating the support-attached dry hydrogel to produce a support-attached vitrigel membrane;
(3) redrying the support-attached vitrigel membrane to remove free water and produce a vitrified support-attached dried vitrigel membrane in the container;
(4) rehydrating and detaching the support-attached dried vitrigel membrane from the container, and mounting the detached membrane on a film;
(5) adhesively securing the rehydrated support-attached vitrigel membrane layered on the film to one open end surface of a tubular frame with a urethane-based adhesive or with double-sided tape or by heat welding;
(6) drying the layered support-attached vitrigel membrane on the film to produce a support-attached dried vitrigel membrane layered on the film;
(7) shaping the layered support-attached dried vitrigel membrane on the film in substantially the same shape as the open end surface of the frame; and
(8) removing the film from the support-attached dried vitrigel membrane.

The steps (1) to (3) of Fourth Embodiment can be performed in the same manner as in Second Embodiment.

In step (4) of Fourth Embodiment, the support-attached vitrigel membrane rehydrated and detached from the container is placed on the film. The film may be appropriately selected from those detachable from the dried vitrigel membrane after the drying of the vitrigel membrane, as in First Embodiment.

In step (5), the rehydrated support-attached vitrigel membrane layered on the film is adhesively secured to one open end surface of the tubular frame. The membrane may be adhesively secured by using, for example, an acrylic double-sided tape. It is preferable in this case to process the double-sided tape according to the shape and size of the end surface of the frame.

The dry membrane obtained in step (6) after the drying of the support-attached vitrigel membrane layered on the film is shaped in substantially the same shape as the open end surface of the frame in step (7). The single cell-culture chamber can then be obtained in step (8) upon removing the film. The drying of the support-attached vitrigel membrane in step (6) can be performed in the same manner as in First to Third Embodiments.

The double cell-culture chamber can be produced as follows. The tubular frame of a single cell-culture chamber produced by using any of the methods described in Second to Fourth Embodiment is brought into contact with the open end surface of another tubular frame from the side of the surface of the support-attached dried vitrigel membrane not adhering to the tubular frame of the single cell-culture chamber. The frames are then joined and fixed with the support-attached dried vitrigel membrane interposed between the two tubular frames to produce the double cell-culture chamber.

### Examples

The present invention is described below in greater detail using Examples. It should be noted, however, that the present invention is in no way limited by the following Examples.

### <Example 1> Production of Collagen Dried vitrigel membrane (Collagen Amount: 0.52 to 2.1 mg/cm²) Adsorbed to Parafilm

The bottom surface of a hydrophobic polystyrene culture Petri dish (Falcon # 35-1007; diameter, 60 mm) was used as the substrate. An acrylic (outer circle diameter: 39 mm; inner circle diameter: 35 mm; height: 10.0 mm) was used as the wall surface mold. The parafilm (Pechiney Plastic Packaging) was used after being cut into a circular shape with a diameter of 50 mm. Note that the wall surface mold and the parafilm were sterilized with a spray of 70% ethanol, and used after wiping off the ethanol. Specifically, the bottom surface of the hydrophobic polystyrene culture Petri dish having a diameter of 60 mm was covered with a circular sheet of parafilm having a diameter of 50 mm, and the wall surface mold was placed thereon to produce a container equipped with the wall surface mold separable from the parafilm covering the substrate.

The collagen gel was produced by injecting a collagen sol (2.0-, 4.0-, 6.0-, or 8.0-ml 0.25% collagen sol) into the container, and allowing the collagen sol to gelate in a 37.0°C humid incubator in the presence of 5.0% CO₂/95% air after placing a lid on the Petri dish. The injected collagen sol gelated without flowing through the gaps between the wall surface mold and the parafilm covering the substrate.

After 4, 6, and 8 hours from the transfer into the 37.0°C humid incubator, the amount of the free water that flowed out of the collagen gel through the gaps between the wall surface mold and the parafilm covering the substrate was quantified. The free water flown out at each time point was removed. At the two-hour period, the wall surface mold was slightly moved up and down to release the adhesion between the collagen gel and the wall surface mold. As a result, 1/3 or more of the free water flowed out of the wall surface mold by the four-hour period in the collagen gels derived from the 6.0 ml and 8.0 ml collagen sols. By the six-hour period, about 1/3 of the free water flowed out in the collagen gel derived from the 4.0 ml collagen sol. About 1/4 of the free water flowed out by the eight-hour period in the collage gel derived from the 2.0 ml collagen sol.

The wall surface mold was removed from the substrate at the eight-hour period. Here, the wall surface mold did not adhere to the collagen gel, and there was no adhesion of the collagen gel to the surrounding areas, including the inner wall of the wall surface mold removed from the parafilm covering the substrate. At the eight-hour period, the collagen gel was transferred from the 37.0°C humid incubator to a clean bench under 10.0°C, 40% humidity conditions. With the lid of the Petri dish removed, the free water remaining in the collagen gel was completely removed by natural drying while allowing an outflow of the free water. As a result, a dry collagen gel was obtained.

Vitrification starts after the remaining free water in the collagen gel is completely removed. The time required to naturally dry the collagen gel before the vitrification (the time before the dry collagen gel is formed after the complete removal of the remaining free water in the collagen gel) was thus measured to give a rough estimate. The time required to start vitrification was 20 hours or less in the 2.0-ml 0.25% collagen gel, and between 20 hours and 41 hours in the 4.0-ml, 6.0-ml, and 8.0-ml 0.25% collagen gels.

The dried collagen gel, 1 to 2 days after the vitrification, was transferred to a clean bench maintained at room temperature, and 5.0-ml PBS was added to the substrate Petri dish to rehydrate the gel and produce a collagen vitrigel membrane adsorbed on the parafilm covering the substrate. The membrane was rinsed several times with 5.0-ml PBS, and a collagen vitrigel membrane equilibrated with the PBS and adsorbed on the parafilm was obtained. The collagen vitrigel membrane reflected the shape of the inner circle of the wall surface mold (diameter: 35 mm; area: 9.6 cm²), and did not have amorphous outer peripheral edge.

The collagen vitrigel membrane adsorbed to the parafilm was then transferred to a hydrophobic polystyrene culture Petri dish (diameter: 60 mm; Falcon # 35-1007), and completely dried by being left unattended for about 1 to 2 days in an open clean bench under 10.0°C, 40% humidity conditions, with the lid removed. The product was transferred to a clean bench maintained at room temperature, and aseptically kept at room temperature to vitrify in the Petri dish with the lid closed. As a result, a collagen dried vitrigel membrane adsorbed on the parafilm was obtained. The collagen dried vitrigel membrane adsorbed to the parafilm was easy to cut into the desired fine shape with scissors, a surgical knife, or the like. It was also easy to detach the collagen dried vitrigel membrane from the parafilm.

Inserting a cyclic nylon film into the collagen sol in the foregoing step can produce a ring-shaped nylon membrane support-attached collagen dried vitrigel membrane adsorbed to the parafilm. Further, by modifying the bottom surface shape and height of the wall surface mold, a collagen dried vitrigel membrane of any shape and thickness can be produced by being adsorbed to the parafilm.

### <Example 2> Production of Cell Culture Chamber

Conventional dried vitrigel membranes are produced by adhering to the culture Petri dish, and cannot be handled in the membrane state. For example, attempts have been made to produce a chamber in which a vitrigel membrane with moisture is fixed by being physically held in a two-phase container such as a Permcell. However, the production involves complicated procedures, and mass production has been difficult.

The present invention establishes the dried vitrigel membrane producing method as above, and enables cell culture chamber production using the dried vitrigel membrane, as follows.

### (1) Single Cell-Culture Chamber

A urethane-based adhesive was applied to one open end surface of an acrylic cylindrical tube, and contacted to the collagen dried vitrigel membrane layered on the parafilm. A weight was placed on the cylindrical tube to ensure adhesion between the two. The cylindrical tube and the collagen dried vitrigel membrane adhering to each other were then adhesively secured by being left unattended at room temperature under properly vented conditions. The portion of the collagen dried vitrigel membrane outwardly protruding from the cylindrical tube was cut to match the shape of the membrane to the end surface of the cylindrical tube. Then, the parafilm was detached from the collagen dried vitrigel membrane adhesively secured to the tube to produce a single cell-culture chamber having the collagen dried vitrigel membrane on the bottom surface.

### (2) Double Cell-Culture Chamber

A single cell-culture chamber (first chamber) produced by using the foregoing method was contacted to another cylindrical tube (second chamber) of the same diameter and a lower height from the side of the surface of the dried collagen vitrigel not adhering to the tubular frame of the single cell-culture chamber. The contact portion was then covered with a parafilm from outside to join and fix the two frames with the dry collagen vitrigel therebetween. In this way, a double cell-culture chamber was produced that had two chambers (first and second chambers) formed via the collagen dried vitrigel membrane.

### <Example 3> Permeability of Protein Membrane of Vitrigel Chamber

The single cell-culture chamber was used. In the following (and in Examples 4 and 6), the chamber of the present invention will be called "vitrigel chamber" to conveniently distinguish it from the commercially available chambers (Comparative Examples below).

### (1) Example

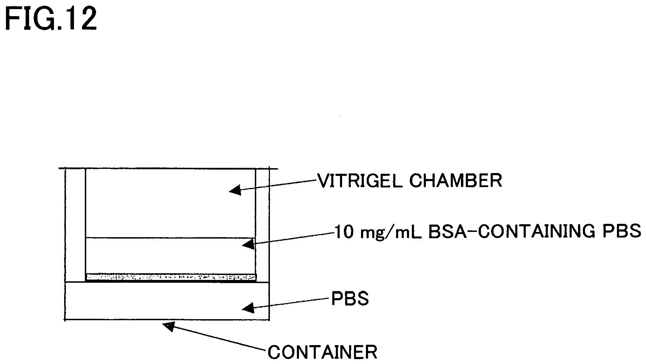

With the vitrigel chamber hung and held inside the container, a phosphate buffered saline (PBS; 500 µl) containing 10 mg/ml BSA, and PBS (1 ml) were injected into the vitrigel chamber and the container, respectively (FIG. 12). The whole was left unattended in a humid incubator (37.0°C, 5% CO₂/95% air) for 16 hours, and the BSA concentration in the PBS inside the container was measured with a Quick Start protein assay kit (Bio-Rad Laboratories; #500-0201JA). The BSA concentration was 2.1 mg/ml, and the BSA passed through the vitrigel membrane.

### (2) Comparative Example

The same test as conducted for the vitrigel chamber in (1) was conducted to examine the protein permeability of a commercially available collagen membrane chamber (Koken: permeable collagen membrane for cell culture #CM-24). (Note, however, that, because the chamber size was smaller than the vitrigel chamber, the liquid amounts in the chamber and the container were adjusted to 337 µl and 674 µl, respectively, to provide the same liquid level in the chamber and the same liquid amount ratio inside and outside of the chamber as those of the vitrigel chamber.) The BSA concentration in the PBS inside the container was at or below the lower detection limit of the kit, and the BSA did not pass through the collagen membrane of the commercially available product.

### <Example 4> Protein Permeability of Vitrigel Chamber (Neurite extension of PC-12 Cells by the Effect of NGF Through Membrane)

### (1) Example

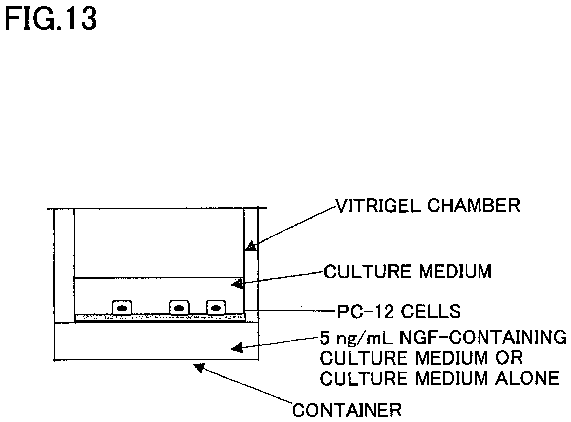

The vitrigel chamber was hung and held in the container (FIG. 5, (A) and (B)), and PC-12 cells suspended in a culture medium [Dulbecco's modified Eagle's culture medium (GIBCO BRL #11885-084) containing 10% inactivated fetal bovine serum (Sigma #F2442), 20 mM HEPES (GIBCO BRL #15630), 100 units/ml penicillin, and 100 µg/ml streptomycin] were seeded in 2.5 × 10³ cells/cm² in the vitrigel chamber. A culture medium (1.2 ml), either containing NGF (upstate #01-125; 5 ng/ml) or by itself, was also injected into the container (FIG. 13). This was statically cultured for 2 days in a humid incubator (37.0°C, 5% CO₂/95% air), and the cell morphology was observed through a phase-contrast microscope.

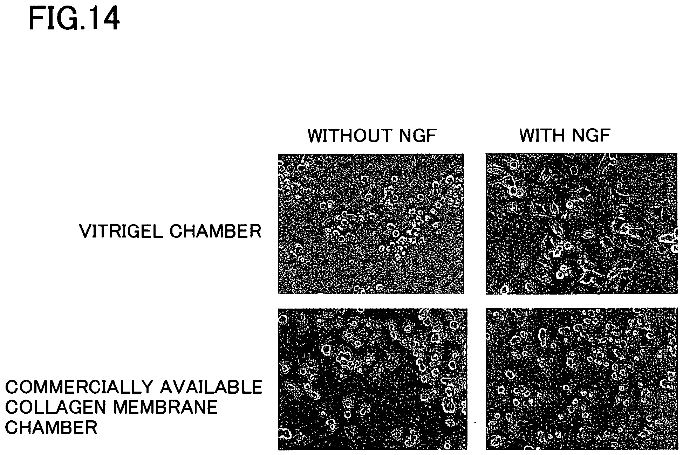

The PC-12 cells were spherical in shape in samples in which only the culture medium was injected into the container, and there was no neurite extensions. On the other hand, neurite extension was confirmed in samples in which the NGF-added culture medium was injected into the container (upper column in FIG. 14). Specifically, the effect of the NGF in the container on the PC-12 cells was confirmed.

### (2) Comparative Example

The same experiment was conducted with the commercially available collagen membrane chamber (Koken; permeable collagen membrane #CM-24 for cell culture). The PC-12 cells were spherical in shape, and neurite extension was not confirmed, irrespective of the presence or absence of NGF (lower column in FIG. 14). Specifically, the NGF in the container did not act on the PC-12 cells.

### <Example 5> Construction of Tissue Model

### (1) Tissue Model Using Single Cell-Culture Chamber

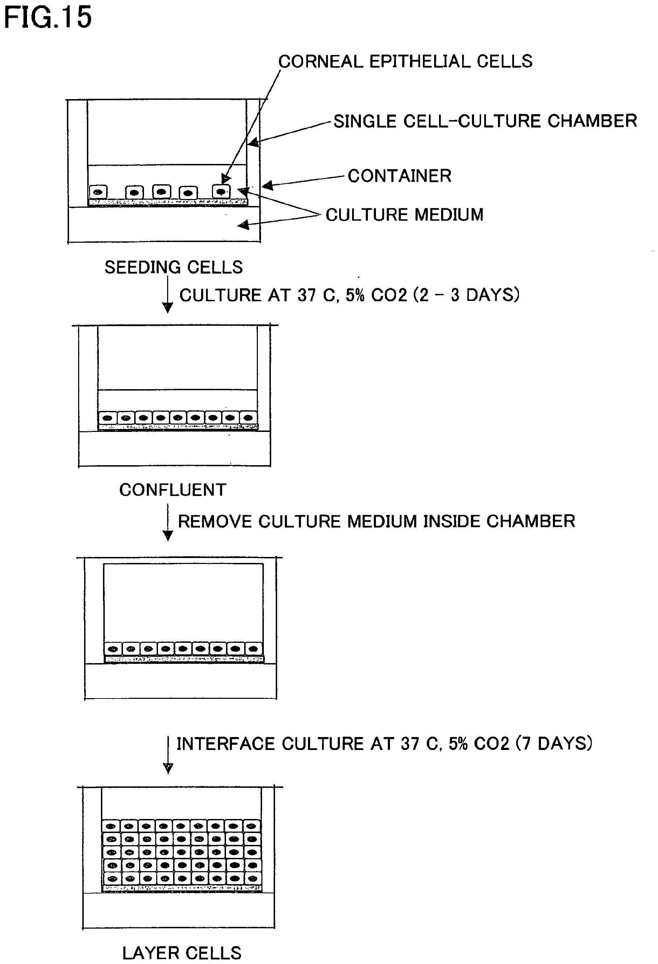

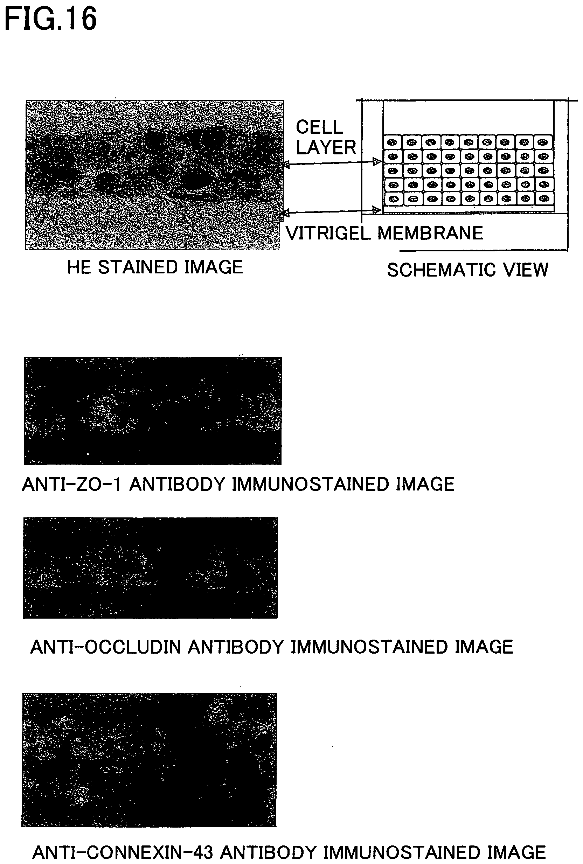

FIG. 15 is a diagram schematically representing the steps of constructing a tissue model using the single cell-culture chamber. In this example, the single cell-culture chamber shown in FIG. 4, (A) and (B) was used. FIG. 16 is a diagram representing stained images of frozen sections of a cultured cornea model.

The single cell-culture chamber was held inside the wells of a 12-well culture plate (Millipore) with the stoppers provided on the frame, and human corneal epithelium cells (6 × 10⁴ cells) suspended in a culture medium (500 µl) were seeded on the dry collagen vitrigel on the bottom surface of the chamber. A culture medium (600 µl) was injected into the wells. The cells were statically cultured to confluence for 2 to 3 days in a humid incubator (37.0°C, 5% CO₂/95% air), and cultured at the liquid-gas interface at 37.0°C in 5% CO₂ for 7 days after removing the culture medium in the chamber. After the interface culture, the cell layer was cross sectioned to produce frozen sections, and the nucleus was dyed with Hoechst 33342 for fluorescence microscope observation. The microscopy confirmed formation of approximately five layers of cells. From day 2 to day 7 of the interface culture, a time-course increase of transepithelial electrical resistance (TEER) value, indicative of formation of cell-to-cell adhesion, was confirmed.

Further, as shown in FIG. 16, immunostaining of the tight junction marker protein (ZO-1, Occludin) and the gap junction marker protein (Connexcin-43) with antibodies revealed expression of these proteins, confirming formation of the tight junction and gap junction seen in corneal epithelium. It was therefore confirmed that the single cell-culture chamber of the present invention can be used to easily construct a human corneal epithelium model.

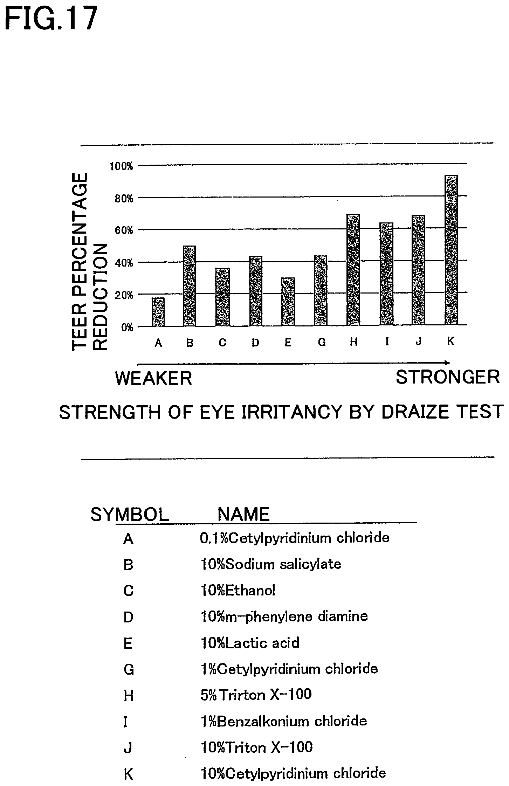

FIG. 17 is a diagram representing the result of the evaluation of eye irritant substances using the human corneal epithelium model. The cell layer surface of the human corneal epithelium model was exposed to eye irritant substances, and time-dependent changes of transepithelial electrical resistance (TEER) were measured. It was found as a result that the TEER had the tendency to decrease more prominently with increase in the stimulus of the eye stimulating substances. Particularly, the TEER percentage reduction after 10 seconds from exposure to the eye irritant substances correlated with the result (Draize scores) of an eye stimulation test (Draize test) using rabbits as test animals. The result suggests that the cornea model constructed with the single cell-culture chamber can be used for the eye stimulation evaluation of compounds as an alternative method of an animal experiment using rabbits.

### (2) Tissue Model Using Double Cell-Culture Chamber

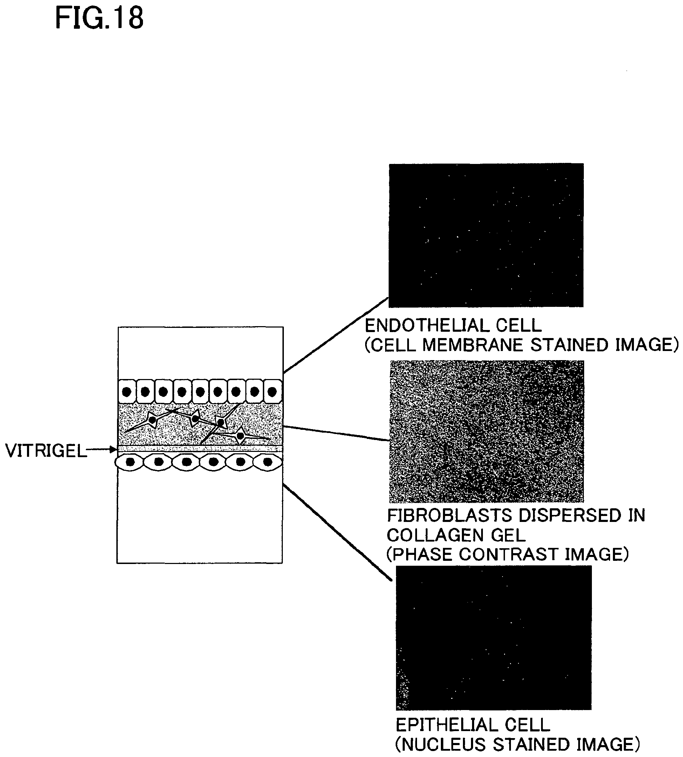

FIG. 18 represents photographs of cells in each layer corresponding to the example represented in the schematic view of FIG. 11.

In order to improve tissue model stability, a cyclic nylon film (1-mm wide) of substantially the same size as the inner diameter of the chamber was placed on the dry collagen vitrigel inside the first chamber of the double cell-culture chamber produced by using the foregoing method.

A collagen sol suspending dermal fibroblasts was seeded in the first chamber 11 (in a thickness of 2 mm), and the cells were statically cultured in a humid incubator (37.0°C, 5% CO₂/95% air) for 2 hours to gelate. After adding a culture medium (200 µl) to the collagen gel surface, the cells were statically cultured in the humid incubator (37.0°C, 5% CO₂/95% air) for 1 day to extend the dermal fibroblasts C1 dispersed in the collagen gel. Then, endothelium cells were seeded on the surface of the collagen gel, and statically cultured to confluence in a humid incubator (37.0°C, 5% CO₂/95% air) for 1 to 2 days.

Thereafter, the double cell-culture chamber Y was inverted, and epithelium cells (dermal keratinocytes) were seeded in the second chamber 12. The epithelium cells were statically cultured to confluence in the humid incubator (37.0°C, 5% CO₂/95% air) for 1 to 2 days. After exchanging the culture medium with a medium that promotes differentiation of the dermal keratinocytes, the cells were statically cultured in the humid incubator (37.0°C, 5% CO₂/95% air) for 2 days. After removing the culture medium, the cells were cultured at the liquid-gas interface to differentiate the epithelium cells.

Observation of the tissue model cross sections revealed that the epithelium cells, the dermal fibroblasts, and the endothelium cells were three-dimensionally layered, as shown in FIG. 18, confirming that the double cell-culture chamber Y can be used to easily construct a skin model.

### <Example 6> Production of Frozen section of Corneal Epithelium Model Produced on Vitrigel Chamber

### (1) Example

The vitrigel membrane (adhering to a corneal epithelium cell layer) of the corneal epithelium model (Example 5(1)) produced on the vitrigel chamber was cut from an acrylic cylinder with a surgical knife. The vitrigel membrane was embedded in a Tissue-Tek O.C.T. compound (Sakura Finetek), and frozen. The frozen sample was then sliced in 5-µm thicknesses in a Cryostat (LEICA CM3050S). The slices contained a (artifact-free) cross-section in which the cell layer adhered to the vitrigel membrane (HE stained image and immunostained image of the slice are shown in FIG. 16).

### (2) Comparative Example

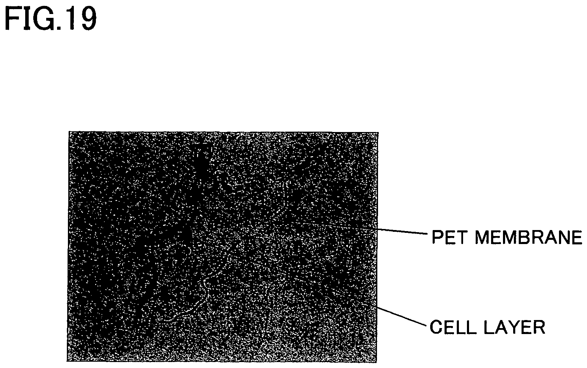

A corneal epithelium model was produced in a commercially available PET membrane chamber (Millipore) by using the same procedures as those used for the vitrigel chamber. Frozen sections were produced by using the same procedure used for the vitrigel chamber. However, the sample cracked at the PET membrane portion and the cell layer detached from the PET membrane, and a (normal) slice with the PET membrane adhering to the cell layer was not obtained (FIG. 19).

### <Example 7> Production of Ring-shaped nylon membrane support-Attached Collagen Dried vitrigel membrane Using Culture Petri Dish

A dried vitrigel membrane not adsorbed to the film was produced through the steps A to C below. Note that the production of the ring-shaped nylon membrane support-attached collagen vitrigel membrane in the steps below is based on the methods of WO2005/014774 and JP-A-2007-185107 filed by the present inventors.

Step A: A single ring-shaped nylon membrane support having an outer circle diameter of 33 mm and an inner circle diameter of 24 mm was inserted into a hydrophobic polystyrene culture Petri dish (Falcon #35-1008; diameter, 35 mm). A 0.25% collagen sol (2.0 ml) was immediately injected into the dish, and, with the lid placed on the Petri dish, the collagen sol was allowed to gelate in a 37.0°C humid incubator in the presence of 5.0% CO₂/95% air. As a result, a collagen gel was produced.

After 2 hours, the gel was transferred from the 37.0°C humid incubator to a clean bench under 10.0°C, 40% humidity conditions. Then, with the lid removed from the Petri dish, the remaining free water in the collagen gel was completely removed by natural drying to obtain a vitrified dry collagen gel.

Vitrification starts after the remaining free water in the collagen gel is completely removed. The dry collagen gel, 1 to 2 days after the vitrification, was transferred to a clean bench maintained at room temperature, and rehydrated with PBS (2.0 ml) added to the Petri dish. The gel was then detached from the bottom and wall surfaces of the Petri dish to produce a ring-shaped nylon membrane support-attached collagen vitrigel membrane. After being rinsed several times with 2.0-ml PBS, a ring-shaped nylon membrane support-attached collagen vitrigel membrane equilibrated with the PBS was produced.

The ring-shaped nylon membrane support-attached collagen vitrigel membrane was completely dried by being left unattended for about 1 to 2 days in a clean bench under 10.0°C, 40% humidity conditions, with the lid removed. The product was transferred to a clean bench maintained at room temperature, and, with the lid placed on the Petri dish, aseptically kept at room temperature to vitrify. As a result, a ring-shaped nylon membrane support-attached collagen dried vitrigel membrane adhering to the Petri dish was produced.

Step B: The product was rehydrated with the PBS (2.0 ml) added to the Petri dish, and traced along the inner wall of the Petri dish with sharp-ended tweezers to detach the ring-shaped nylon membrane support-attached collagen vitrigel membrane from the bottom and wall surfaces of the Petri dish.

Step C: The ring-shaped nylon membrane support-attached collagen vitrigel membrane was held between two circular magnets (outer circle diameter: 33 mm; inner circle diameter: 24 mm; thickness: 1 mm), and allowed to dry by being left unattended overnight in a clean bench under 10.0°C, 40% humidity conditions. As a result, a collagen dried vitrigel membrane held between the circular magnets was produced.

### <Example 8> Production of Ring-shaped nylon membrane support-Attached Collagen Dried vitrigel membrane Using Wall surface mold

A dried vitrigel membrane not adsorbed to the film was produced through the steps A to C below.

Step A: The bottom surface of a hydrophobic polystyrene culture Petri dish (245 × 245 mm) was used as the substrate, and 34 acrylic wall surface molds (outer circle diameter: 38 mm; inner circle diameter: 34 mm; height: 30 mm) were used. The 34 wall surface molds were placed on the single substrate to produce 34 containers equipped with the wall surface molds separable from the substrate. A single sheet of a ring-shaped nylon membrane support was inserted into each container, and a 0.25% collagen sol. (2.0 ml) was injected into the container. After placing a lid on the substrate Petri dish, the collagen sol was allowed to gelate in a 37.0°C humid incubator in the presence of 5.0% CO₂/95% air. As a result, 34 collagen gels were produced on the single substrate.

At the two-hour period, the wall surface molds were slightly moved up and down to release the adhesion between the collagen gels and the wall surface molds. By the four- to six-hour period, about 1/3 of the free water flowed out of the wall surface molds, and the wall surface molds were separated from the substrate. After removing the discharged free water, the gels were transferred to a clean bench under 10.0°C, 40% humidity conditions. With the lid of the Petri dish removed, the remaining free water in the collagen gels was completely removed by naturally drying the gels for 2 days, and dry collagen gels were obtained.

Vitrification starts after the remaining free water in the collagen gel is completely removed. The dry collagen gel, 1 to 2 days after the vitrification, was transferred to a clean bench maintained at room temperature, and rehydrated with the PBS (100 ml) added to the Petri dish. The gel was then detached from the bottom and wall surfaces of the Petri dish to produce a ring-shaped nylon membrane support-attached collagen vitrigel membrane.

With the lid removed, the ring-shaped nylon membrane support-attached collagen vitrigel membrane was completely dried by being left unattended in a clean bench for about 1 to 2 days under 10.0°C, 40% humidity conditions, and transferred to a clean bench maintained at room temperature. With the lid placed on the Petri dish, the product was aseptically kept at room temperature to vitrify, and a ring-shaped nylon membrane support-attached collagen dried vitrigel membrane adhered to the Petri dish was produced.

Step B: The 34 ring-shaped nylon membrane support-attached collagen vitrigel membranes were rehydrated with the PBS (100 ml) added to the Petri dish, and detached from the bottom and wall surfaces of the Petri dish.

Step C: Each ring-shaped nylon membrane support-attached collagen vitrigel membrane was held between two circular magnets (outer circle diameter: 33 mm; inner circle diameter: 24 mm; thickness: 1 mm), and dried by being left unattended overnight in a clean bench under 10.0°C, 40% humidity conditions to produce 34 collagen dried vitrigel membranes held between the circular magnets.

### <Example 9> Adhesively Securing Collagen Dried vitrigel membrane (Examples 7 and 8) to Tubular Frame

The collagen dried vitrigel membranes produced in Examples 7 and 8 were adhesively secured to the tubular frame.

### a) Adhesive Securing with Urethane-Based Adhesive

A urethane-based adhesive (Cemedine, No. UM700) was applied to one open end surface of an acrylic cylindrical tube (outer diameter: 15 mm; inner diameter: 11 mm). The collagen dried vitrigel membrane produced in Examples 7 and 8 and held between the circular magnets was then contacted to the adhesive to adhesively secure the membrane. The portion of the collagen dried vitrigel membrane outwardly protruding from the cylindrical tube between the circular magnets was cut to match the shape of the membrane with the end surface shape of the cylindrical tube, and a single cell-culture chamber having the collagen dried vitrigel membrane on the bottom surface was produced.

In addition to the single culture chamber, it was also possible to produce a double culture chamber by using the same method.

### b) Adhesive Securing Using Double-Sided Tape

An acrylic adhesion double-sided tape (Nitto Denko Corporation, No.57115B) cut in the same size as the end surface of an acryl cylindrical tube (outer diameter: 15 mm; inner diameter: 11 mm) was attached to one open end surface of the cylindrical tube, and the collagen dried vitrigel membrane produced in Examples 7 and 8 and held between circular magnets was attached to the tape to adhesively secure the membrane. The portion of the collagen dried vitrigel membrane outwardly protruding from the cylindrical tube between the circular magnets was cut to match the shape of the membrane with the end surface shape of the cylindrical tube, and a single cell-culture chamber having the collagen dried vitrigel membrane on the bottom surface was produced.

In addition to the single culture chamber, it was also possible to produce a double culture chamber by using the same method.

### c) Adhesive Securing by Heat welding

The collagen dried vitrigel membrane produced in Examples 7 and 8 and held between circular magnets was contacted to one open end surface of a polystyrene or acrylic cylindrical tube (outer diameter: 15 mm; inner diameter: 11 mm), and only the contact portion was heated with a heat sealer to adhesively secure (heat fuse) the membrane. The portion of the collagen dried vitrigel membrane outwardly protruding from the cylindrical tube between the circular magnets was cut to match the shape of the membrane with the end surface shape of the cylindrical tube, and a single cell-culture chamber having the collagen dried vitrigel membrane on the bottom surface was produced.

In addition to the single culture chamber, it was also possible to produce a double culture chamber by using the same method.

### <Example 10> Cell Culture Chamber Production by Drying Film-Adsorbed Vitrigel Membrane in Contact with Tubular Frame for Adhesive Securing

A cell culture chamber with the vitrigel membrane adhesively secured to the tubular frame was produced through the steps A to D below.

Note that the production of the ring-shaped nylon membrane support-attached collagen vitrigel membrane in the following steps is based on the method of WO2005/014774 filed by the present inventors.

Step A: A single sheet of a ring-shaped nylon membrane support (outer circle diameter: 33 mm; inner circle diameter: 24 mm) was inserted into a hydrophobic polystyrene culture Petri dish (Falcon #35-1008; diameter, 35 mm). A 0.25% collagen sol (2.0 ml) was immediately injected into the dish, and, with the lid placed on the Petri dish, the collagen sol was allowed to gelate in a 37.0°C humid incubator in the presence of 5.0% CO₂/95% air. As a result, a collagen gel was produced.

After 2 hours, the gel was transferred from the 37.0°C humid incubator to a clean bench under 10.0°C, 40% humidity conditions. Then, with the lid removed from the Petri dish, the remaining free water in the collagen gel was completely removed by natural drying to obtain a vitrified dry collagen gel.

Vitrification starts after the remaining free water in the collagen gel is completely removed. The dry collagen gel, 1 to 2 days after the vitrification, was transferred to a clean bench maintained at room temperature, and rehydrated with PBS (2.0 ml) added to the Petri dish. The gel was then detached from the bottom and wall surfaces of the Petri dish to produce a ring-shaped nylon membrane support-attached collagen vitrigel membrane. After being rinsed several times with 2.0-ml PBS, a ring-shaped nylon membrane support-attached collagen vitrigel membrane equilibrated with the PBS was produced.

With the lid removed, the ring-shaped nylon membrane support-attached collagen vitrigel membrane was completely dried by being left unattended for about 1 to 2 days in a clean bench under 10.0°C, 40% humidity conditions. The product was transferred to a clean bench maintained at room temperature, and, with the lid placed on the Petri dish, aseptically kept at room temperature to vitrify. As a result, a ring-shaped nylon membrane support-attached collagen dried vitrigel membrane adhering to the Petri dish was produced.

Step B: The product was rehydrated with the PBS (2.0 ml) added to the Petri dish, and traced along the inner wall of the Petri dish with sharp-ended tweezers to detach the ring-shaped nylon membrane support-attached collagen vitrigel membrane from the bottom and wall surfaces of the Petri dish.

Step C: The bottom surface of a hydrophobic polystyrene culture Petri dish (diameter, 150 mm) was covered with a single polyethylene sheet, and the rehydrated ring-shaped nylon membrane support-attached collagen vitrigel membrane was placed thereon. An acrylic adhesion double-sided tape (Nitto Denko Corporation, No.57115B) cut in the same size as the end surface of an acryl cylindrical tube was attached to one open end surface of the cylindrical tube, and the ring-shaped nylon membrane support-attached collagen vitrigel membrane layered on the polyethylene sheet was contacted to the open end surface of the cylindrical tube. Then, a weight was placed on the cylindrical tube, and the whole was left unattended until the vitrigel membrane dried in the clean bench to adhesively secure the two.

Step D: The cylindrical tube adhering to the ring-shaped nylon membrane support-attached collagen dried vitrigel membrane layered on the polyethylene sheet was completely dried by being left unattended in a clean bench for about 1 day under 10.0°C, 40% humidity conditions. Then, the portion of the collagen dried vitrigel membrane outwardly protruding from the cylindrical tube was cut to match the shape of the membrane with the end surface shape of the cylindrical tube. The polyethylene sheet was then detached from the adhesively secured collagen dried vitrigel membrane, and a single cell-culture chamber having the collagen dried vitrigel membrane on the bottom surface was produced.

In addition to the single culture chamber, it was also possible to produce a double culture chamber by using the same method.

### <Example 11> Tissue Model (Organoid Plate Constructed from Two Collagen Vitrigel Membranes and Three Cell Types) Producing Method Using Double Cell-Culture Chamber

Step A: Human dermal fibroblasts (1 × 10⁴ cells) suspended in 500 µl of a culture medium (DMEM, 10% FBS, 20 mM HEPES, 100 units/ml penicillin, 100 µg/ml streptomycin, 0.1 mM l-ascorbic acid phosphate, magnesium salt n-hydrate) were seeded on the collagen dried vitrigel membrane in the second chamber of the double cell-culture chamber. The cells were statically cultured in a humid incubator (37.0°C, 5% CO₂/95% air) for 8 days to induce collagen production and multilayer cell layer formation in the fibroblasts.

Step B: A human corneal epithelium cell layer was constructed in the single culture chamber according to the method described in Example 5(1). Specifically, human corneal epithelium cells (6 × 10⁴ cells) suspended in a culture medium (500 µl) were seeded on the collagen vitrigel membrane on the bottom surface of the chamber, and statically cultured in a humid incubator (37.0°C, 5% CO₂/95% air) for 2 to 3 days. After removing the culture medium inside the chamber, the cells were cultured at the liquid-gas interface in a humid incubator (37.0°C, 5% CO₂/95% air) for 7 days. As a result, approximately five layers of cells were formed.

Step C: An acrylic cylindrical frame of the same diameter as the chamber and a lower height (outer diameter: 15 mm; inner diameter: 11 mm; height: 5 mm) was attached to the surface (outer side) of the single cell-culture chamber (containing the human corneal epithelium cell layer constructed therein; produced in step B) not adhering to the cells. As a result, a culture chamber of substantially the same shape as the double cell-culture chamber was produced.

The cell culture chamber was inverted, and human dermal fibroblasts (500 µl) prepared by using the same procedures used in step 1 were seeded in the newly formed second chamber. The cells were then statically cultured in a humid incubator (37.0°C, 5% CO₂/95% air) for 1 day.

Step D: The cell culture chamber was inverted again, and the acrylic frame temporarily fixed for the formation of the second chamber was removed. The collagen vitrigel membrane was then cut along the inner wall of the chamber with a dissecting surgical knife. As a result, a collagen vitrigel membrane was produced in which the human corneal epithelium cells were formed on one side, and the human dermal fibroblast layer on the other side. The membrane was layered on the second-chamber side of the cell culture chamber of step 1 with the human dermal fibroblast layer side facing the human dermal fibroblast layer produced in step 1. The cells were statically cultured in a humid incubator (37.0°C, 5% CO₂/95% air) for 3 days to fuse the first human dermal fibroblast layer with the second human dermal fibroblast layer. As a result, a model was produced in which the collagen vitrigel membrane, the human dermal fibroblast layer, the collagen vitrigel membrane, and the human corneal epithelium cells were laminated in order in the second chamber of the double culture chamber.

Step E: The double cell-culture chamber produced in step D was inverted, and the acrylic frame fixed for forming the second chamber was removed to restore the form of the single cell-culture chamber. The cell culture chamber was held in the wells of a 12-well culture plate with the stoppers provided on the frame, and human skin microvascular endothelial cells (8 × 10⁴ cells) suspended in a culture medium (500 µl) were seeded on the collagen vitrigel membrane on the bottom surface of the chamber well corresponding to a single chamber. The cells were then statically cultured in a humid incubator (37.0°C, 5% CO₂/95% air) for 1 day.

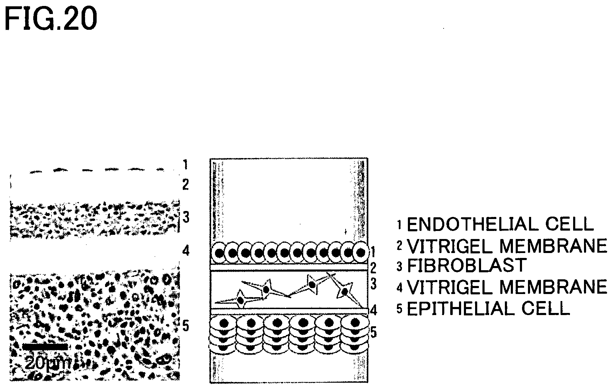

Step F: Observation of the tissue model cross section confirmed that the human corneal epithelium cell layer, the collagen vitrigel membrane, the human dermal fibroblast layer, the collagen vitrigel membrane, and the human skin microvascular endothelial cells were three-dimensionally laminated as shown in FIG. 20. It was therefore confirmed that the organoid plate mimicking epithelium, mesenchyme, and endothelium can easily be constructed.

### Reference Signs List

- 1: Tubular frame
- 2: Dried vitrigel membrane
- 3: Film
- 4: Stopper
- X: Single Cell-Culture Chamber
- Y: Double Cell-Culture Chamber

## Claims

1. A single cell-culture chamber comprising a dried vitrigel membrane covering and secured to one open end surface of a tubular frame, wherein the dried vitrigel membrane is secured to the open end surface of the frame:
with a urethane-based adhesive; or
with double-sided tape; or
by heat welding.

2. The single cell-culture chamber of claim 1, wherein the dried vitrigel membrane has substantially the same shape as the open end surface of the frame.

3. The single cell-culture chamber of claim 1 or claim 2, wherein the frame includes an outwardly protruding stopper provided on an outer periphery portion of the open end surface opposite from the end surface covered by and secured to the dried vitrigel membrane.

4. A double cell-culture chamber comprising two tubular frames joined and secured to each other with a dried vitrigel membrane interposed between the opposing open end surfaces of the tubular frames so as to form a first chamber and a second chamber via the dried vitrigel membrane, wherein the dried vitrigel membrane is secured to one open end surface of the frame:
with a urethane-based adhesive; or
with double-sided tape; or
by heat welding.

5. The double cell-culture chamber of claim 4, wherein the dried vitrigel membrane has substantially the same shape as the open end surfaces of the frames.

6. The double cell-culture chamber of either one of claim 4 or claim 5, wherein the two frames are adhesively secured to each other from outside with a film-shaped adhesive material around the opposing open end surfaces.

7. A tissue model constructed from a single- or multi-layer culture of cells seeded on the dried vitrigel membrane inside the single cell-culture chamber of any one of claims 1 to 3.

8. The tissue model of claim 7, wherein the tissue model is any one of a chemical transdermal absorption model, a chemical corneal permeation model, a chemical gastrointestinal absorption model such as in intestinal tract, a chemical airway absorption model such as in lungs, a chemical vascular permeation model, a chemical hepatic metabolism model, a chemical renal glomerular filtration and excretion model, a chemical dermotoxicity evaluation model, a chemical keratotoxicity evaluation model, a chemical oral mucosal toxicity evaluation model, a chemical neurotoxicity evaluation model, a chemical hepatotoxicity evaluation model, a chemical nephrotoxicity evaluation model, a chemical embryogenic toxicity evaluation model, and an angiogenesis model or a cancer infiltration model for drug development.

9. A tissue model producing method comprising the step of seeding one or more types of cells on the dried vitrigel membrane inside the single cell-culture chamber of any one of claims 1 to 3, and culturing the cells in a single layer or multiple layers.

10. A tissue model constructed from a single- or multi-layer culture of cells seeded on both surfaces of the dried vitrigel membrane inside the double cell-culture chamber of any one of claims 4 to 6.

11. The tissue model of claim 10, wherein the tissue model is any one of a chemical transdermal absorption model, a chemical corneal permeation model, a chemical gastrointestinal absorption model such as in intestinal tract, a chemical airway absorption model such as in lungs, a chemical vascular permeation model, a chemical hepatic metabolism model, a chemical renal glomerular filtration and excretion model, a chemical dermotoxicity evaluation model, a chemical keratotoxicity evaluation model, a chemical oral mucosal toxicity evaluation model, a chemical neurotoxicity evaluation model, a chemical hepatotoxicity evaluation model, a chemical nephrotoxicity evaluation model, a chemical embryogenic toxicity evaluation model, and an angiogenesis model or cancer infiltration model for drug development.

12. A method for producing a tissue model inside the double cell-culture chamber of any one of claims 4 to 6,
the method comprising the steps of:
seeding cells on the vitrigel membrane from a first chamber side and culturing the cells in a single layer or multiple layers; and
inverting the culture chamber, and seeding cells on the vitrigel membrane from a second chamber side and culturing the cells in a single layer or multiple layers.

13. A single cell-culture chamber producing method comprising the steps of:
(1) covering a substrate with a film detachably provided for a dried vitrigel membrane and forming a hydrogel inside a wall surface mold placed on the film, said wall surface mold being a tubular frame with no top and bottom surfaces, and allowing a part of the free water inside the hydrogel to flow out through a gap between the substrate and the wall surface mold;
(2) removing the wall surface mold from the substrate;
(3) drying the hydrogel to remove the remaining free water and produce a vitrified dry hydrogel;
(4) rehydrating the dry hydrogel to produce a vitrigel membrane;
(5) redrying the vitrigel membrane to remove free water and produce a vitrified dried vitrigel membrane;
(6) detaching the dried vitrigel membrane adsorbed to the film from the substrate together with the film, and adhesively securing the dried vitrigel membrane side of the film to one open end surface of a tubular frame with a urethane-based adhesive or with double sided tape or by heat welding;
(7) shaping the dried vitrigel membrane in substantially the same shape as the open end surface of the frame; and
(8) removing the film from the dried vitrigel membrane.

14. A single cell-culture chamber producing method comprising the steps of:
(1) forming a support-containing hydrogel in a container, and drying the hydrogel to remove free water and produce a vitrified support-attached dry hydrogel;
(2) rehydrating the support-attached dry hydrogel to produce a support-attached vitrigel membrane;
(3) redrying the support-attached vitrigel membrane to remove free water and produce a vitrified support-attached dried vitrigel membrane in the container;
(4) rehydrating and detaching the support-attached dried vitrigel membrane from the container, and drying the detached membrane held between magnets to produce a support-attached dried vitrigel membrane detached from the substrate;
(5) adhesively securing the support-attached dried vitrigel membrane to one open end surface of a tubular frame with a urethane-based adhesive or with double sided tape or by heat welding after being detached from the substrate; and
(6) shaping the support-attached dried vitrigel membrane in substantially the same shape as the open end surface of the frame.

15. A single cell-culture chamber producing method comprising the steps of:
(1) forming a support-containing hydrogel inside a wall surface mold placed on a substrate, and allowing a part of the free water inside the hydrogel to flow out through a gap between the substrate and the wall surface mold;
(2) removing the wall surface mold from the substrate;
(3) drying the support-containing hydrogel to remove the remaining free water and produce a vitrified support-attached dry hydrogel;
(4) rehydrating the support-attached dry hydrogel to produce a support-attached vitrigel membrane;
(5) redrying the support-attached vitrigel membrane to remove free water and produce a vitrified support-attached dried vitrigel membrane on the substrate;
(6) rehydrating and detaching the support-attached dried vitrigel membrane from the substrate, and drying the membrane held between magnets to produce a support-attached dried vitrigel membrane detached from the substrate;
(7) adhesively securing the support-attached dried vitrigel membrane to one open end surface of a tubular frame with a urethane-based adhesive or with double sided tape or by heat welding after being detached from the substrate; and
(8) shaping the support-attached dried vitrigel membrane in substantially the same shape as the open end surface of the frame.

16. A single cell-culture chamber producing method comprising the steps of:
(1) forming a support-containing hydrogel inside a container, and drying the hydrogel to remove free water and produce a vitrified support-attached dry hydrogel;
(2) rehydrating the support-attached dry hydrogel to produce a support-attached vitrigel membrane;
(3) redrying the support-attached vitrigel membrane to remove free water and produce a vitrified support-attached dried vitrigel membrane in the container;
(4) rehydrating and detaching the support-attached dried vitrigel membrane from the container, and mounting the detached membrane on a film;
(5) adhesively securing the rehydrated support-attached vitrigel membrane layered on the film to one open end surface of a tubular frame with a urethane-based adhesive or with double sided tape or by heat welding;
(6) drying the layered support-attached vitrigel membrane on the film to produce a support-attached dried vitrigel membrane layered on the film;
(7) shaping the layered support-attached dried vitrigel membrane on the film in substantially the same shape as the open end surface of the frame; and
(8) removing the film from the support-attached dried vitrigel membrane.

17. A double cell-culture chamber producing method comprising the step of contacting the tubular frame of the single cell-culture chamber produced by using the method of any one of claims 13 to 16 to an open end surface of another tubular frame of the same planar cross-sectional shape from the surface side not adhering to the adhesively secured dried vitrigel membrane or support-attached dried vitrigel membrane, and joining and securing the two tubular frames to each other with the dried vitrigel membrane or the support-attached dried vitrigel membrane interposed therebetween.

## Patentansprüche

1. Zellkultur-Einzelkammer, die eine getrocknete Vitrigelmembran umfasst, die eine offene Endfläche eines röhrenförmigen Gerüsts bedeckt und daran befestigt ist, wobei die getrocknete Vitrigelmembran an der offenen Endfläche des Gerüsts:
mit einem Klebstoff auf Urethanbasis; oder
mit doppelseitigem Klebeband; oder
mittels Hitzeverschweißen befestigt ist.

2. Zellkultur-Einzelkammer nach Anspruch 1, wobei die getrocknete Vitrigelmembran im Wesentlichen dieselbe Form aufweist wie die offene Endfläche des Gerüsts.

3. Zellkultur-Einzelkammer nach Anspruch 1 oder Anspruch 2, wobei das Gerüst einen nach außen ragenden Anschlag beinhaltet, der an einem Außenumfangsabschnitt der offenen Endfläche gegenüber der Endfläche vorgesehen ist, die von der getrockneten Vitrigelmembran bedeckt wird und daran befestigt ist.

4. Zellkultur-Doppelkammer, die zwei röhrenförmige Gerüste umfasst, die miteinander verbunden und aneinander befestigt sind, wobei eine getrocknete Vitrigelmembran zwischen den gegenüberliegenden offenen Endflächen der röhrenförmigen Gerüste angeordnet ist, um über die getrocknete Vitrigelmembran eine erste Kammer und eine zweite Kammer zu bilden, wobei die getrocknete Vitrigelmembran an einer offenen Endfläche des Gerüsts:
mit einem Klebstoff auf Urethanbasis; oder
mit doppelseitigem Klebeband; oder
mittels Hitzeverschweißen befestigt ist.

5. Zellkultur-Doppelkammer nach Anspruch 4, wobei die getrocknete Vitrigelmembran im Wesentlichen dieselbe Form aufweist wie die offenen Endflächen der Gerüste.

6. Zellkultur-Doppelkammer nach Anspruch 4 oder Anspruch 5, wobei die beiden Gerüste von außen mit einem folienartigen Klebstoff um die gegenüberliegenden offenen Endflächen herum haftend aneinander befestigt sind.

7. Gewebemodell, das aus einer Einzel- oder Mehrfachschichtkultur aus Zellen aufgebaut ist, mit denen die getrocknete Vitrigelmembran in der Zellkultur-Einzelkammer nach einem der Ansprüche 1 bis 3 besiedelt ist.

8. Gewebemodell nach Anspruch 7, wobei das Gewebemodell beliebig ein Modell zur Absorption chemischer Substanzen durch die Haut, ein Modell zur Permeation chemischer Substanzen in die Hornhaut, ein Modell zur Magen-Darm-Absorption chemischer Substanzen, beispielsweise im Magen-Darm-Trakt, ein Modell zur Absorption chemischer Substanzen in den Atemwegen, beispielsweise der Lunge, ein Modell zur Permeation chemischer Substanzen in Gefäße, ein Modell zur Verstoffwechslung chemischer Substanzen in der Leber, ein Modell zur glomerulären Filterung und Exkretion chemischer Substanzen in den Nieren, ein Modell zur Beurteilung der Dermotoxizität chemischer Substanzen, ein Modell zur Beurteilung der Hornhaut-Toxizität chemischer Substanzen, ein Modell zur Beurteilung der Toxizität chemischer Substanzen für die Mundschleimhaut, ein Modell zur Beurteilung der Neurotoxizität chemischer Substanzen, ein Modell zur Beurteilung der Lebertoxizität chemischer Substanzen, ein Modell zur Beurteilung der Nierentoxizität chemischer Substanzen, ein Modell zur Beurteilung der Embryotoxizität chemischer Substanzen oder ein Angiogenesemodell oder ein Krebsinfiltrationsmodell für die Medikamentenentwicklung ist.

9. Verfahren zur Herstellung eines Gewebemodells, das den Schritt des Besiedelns der getrockneten Vitrigelmembran mit einer oder mehreren Arten von Zellen in der Zellkultur-Einzelkammer nach einem der Ansprüche 1 bis 3 und das Kultivieren der Zellen in einer Einzelschicht oder in mehreren Schichten umfasst.

10. Gewebemodell, das aus einer Einzel- oder Mehrfachschichtkultur aus Zellen aufgebaut ist, mit denen beide Oberflächen der getrockneten Vitrigelmembran in der Zellkultur-Doppelkammer nach einem der Ansprüche 4 bis 6 besiedelt sind.

11. Gewebemodell nach Anspruch 10, wobei das Gewebemodell beliebig ein Modell zur Absorption chemischer Substanzen durch die Haut, ein Modell zur Permeation chemischer Substanzen in die Hornhaut, ein Modell zur Magen-Darm-Absorption chemischer Substanzen, beispielsweise im Magen-Darm-Trakt, ein Modell zur Absorption chemischer Substanzen in den Atemwegen, beispielsweise der Lunge, ein Modell zur Permeation chemischer Substanzen in Gefäße, ein Modell zur Verstoffwechslung chemischer Substanzen in der Leber, ein Modell zur glomerulären Filterung und Exkretion chemischer Substanzen in den Nieren, ein Modell zur Beurteilung der Dermotoxizität chemischer Substanzen, ein Modell zur Beurteilung der Hornhaut-Toxizität chemischer Substanzen, ein Modell zur Beurteilung der Toxizität chemischer Substanzen für die Mundschleimhaut, ein Modell zur Beurteilung der Neurotoxizität chemischer Substanzen, ein Modell zur Beurteilung der Lebertoxizität chemischer Substanzen, ein Modell zur Beurteilung der Nierentoxizität chemischer Substanzen, ein Modell zur Beurteilung der Embryotoxizität chemischer Substanzen oder ein Angiogenesemodell oder ein Krebsinfiltrationsmodell für die Medikamentenentwicklung ist.

12. Verfahren zur Herstellung eines Gewebemodells in der Zellkultur-Doppelkammer nach einem der Ansprüche 4 bis 6,
wobei das Verfahren folgende Schritte umfasst:
Besiedeln der Vitrigelmembran mit Zellen von einer ersten Kammerseite aus und Kultivieren der Zellen in einer Einzelschicht oder in mehreren Schichten, und
Umdrehen der Kulturkammer und Besiedeln der Vitrigelmembran mit Zellen von einer zweiten Kammerseite aus und Kultivieren der Zellen in einer Einzelschicht oder in mehreren Schichten.

13. Verfahren zur Herstellung einer Zellkultur-Einzelkammer, das folgende Schritte umfasst:
(1) Bedecken eines Substrats mit einer Folie, die lösbar für eine getrocknete Vitrigelmembran vorgesehen ist, und Bilden eines Hydrogels in einer Wandflächenform, die auf der Folie platziert ist, wobei die Wandflächenform ein röhrenförmiges Gestell ohne obere und untere Fläche ist, und Zulassen, dass ein Teil des freien Wassers in dem Hydrogel durch einen Spalt zwischen dem Substrat und der Wandflächenform herausfließt;
(2) Abnehmen der Wandflächenform vom Substrat;
(3) Trocknen des Hydrogels zum Beseitigen des verbleibenden freien Wassers und Herstellen eines vitrifizierten trockenen Hydrogels;
(4) Rehydrieren des trockenen Hydrogels zum Herstellen einer Vitrigelmembran;
(5) erneutes Trocknen der Vitrigelmembran zum Beseitigen von freiem Wasser und Herstellen einer vitrifizierten getrockneten Vitrigelmembran;
(6) Lösen der getrockneten Vitrigelmembran, die an der Folie adsorbiert ist, von dem Substrat gemeinsam mit der Folie und haftendes Befestigen der die getrocknete Vitrigelmembran aufweisenden Seite der Folie an einer offenen Endfläche eines röhrenförmigen Gerüsts mit einem Klebstoff auf Urethanbasis oder mit einem doppelseitigen Klebeband oder mittels Hitzeverschweißen;
(7) Ausformen der getrockneten Vitrigelmembran in im Wesentlichen derselben Form wie die offene Endfläche des Gerüsts; und
(8) Abnehmen der Folie von der getrockneten Vitrigelmembran.

14. Verfahren zur Herstellung einer Zellkultur-Einzelkammer, das folgende Schritte umfasst:
(1) Bilden eines einen Träger enthaltenden Hydrogels in einem Behälter und Trocknen des Hydrogels zum Beseitigen von freiem Wasser und Herstellen eines vitrifizierten trockenen Hydrogels, das an einem Träger angebracht ist;
(2) Rehydrieren des trockenen Hydrogels, das an einem Träger angebracht ist, zum Herstellen einer Vitrigelmembran, die an einem Träger angebracht ist;
(3) erneutes Trocknen der Vitrigelmembran, die an einem Träger angebracht ist, zum Beseitigen von freiem Wasser und Herstellen einer vitrifizierten getrockneten Vitrigelmembran, die an einem Träger angebracht ist, in dem Behälter;
(4) Rehydrieren und Entfernen der getrockneten Vitrigelmembran, die an einem Träger angebracht ist, aus dem Behälter und Trocknen der entfernten Membran, die zwischen Magneten gehalten wird, damit eine getrocknete Vitrigelmembran hergestellt wird, die an einem Träger angebracht ist und vom Substrat gelöst ist;
(5) haftendes Befestigen der getrockneten Vitrigelmembran, die an einem Träger angebracht ist, an einer offenen Endfläche eines röhrenförmigen Gerüsts mit einem Klebstoff auf Urethanbasis oder mit einem doppelseitigen Klebeband oder mittels Hitzeverschweißen, nachdem sie von dem Substrat gelöst wurde; und
(6) Ausformen der getrockneten Vitrigelmembran, die an einem Träger angebracht ist, in im Wesentlichen derselben Form wie die offene Endfläche des Gerüsts.

15. Verfahren zur Herstellung einer Zellkultur-Einzelkammer, das folgende Schritte umfasst:
(1) Bilden eines einen Träger enthaltenden Hydrogels in einer Wandflächenform, die auf einem Substrat platziert ist, und Zulassen, dass ein Teil des freien Wassers in dem Hydrogel durch einen Spalt zwischen dem Substrat und der Wandflächenform herausfließt;
(2) Abnehmen der Wandflächenform vom Substrat;
(3) Trocknen des einen Träger enthaltenden Hydrogels zum Beseitigen des verbleibenden freien Wassers und Herstellen eines vitrifizierten trockenen Hydrogels, das an einem Träger angebracht ist;
(4) Rehydrieren des trockenen Hydrogels, das an einem Träger angebracht ist, zum Herstellen einer Vitrigelmembran, die an einem Träger angebracht ist;
(5) erneutes Trocknen der Vitrigelmembran, die an einem Träger angebracht ist, zum Beseitigen von freiem Wasser und Herstellen einer vitrifizierten getrockneten Vitrigelmembran, die an einem Träger angebracht ist, auf dem Substrat;
(6) Rehydrieren und Lösen der getrockneten Vitrigelmembran, die an einem Träger angebracht ist, von dem Substrat und Trocknen der Membran, die zwischen Magneten gehalten wird, damit eine getrocknete Vitrigelmembran hergestellt wird, die an einem Träger angebracht ist und vom Substrat gelöst ist;
(7) haftendes Befestigen der getrockneten Vitrigelmembran, die an einem Träger angebracht ist, an einer offenen Endfläche eines röhrenförmigen Gerüsts mit einem Klebstoff auf Urethanbasis oder mit einem doppelseitigen Klebeband oder mittels Hitzeverschweißen, nachdem sie von dem Substrat gelöst wurde; und
(8) Ausformen der getrockneten Vitrigelmembran, die an einem Träger angebracht ist, in im Wesentlichen derselben Form wie die offene Endfläche des Gerüsts.

16. Verfahren zur Herstellung einer Zellkultur-Einzelkammer, das folgende Schritte umfasst:
(1) Bilden eines einen Träger enthaltenden Hydrogels in einem Behälter und Trocknen des Hydrogels zum Beseitigen von freiem Wasser und Herstellen eines vitrifizierten trockenen Hydrogels, das an einem Träger angebracht ist;
(2) Rehydrieren des trockenen Hydrogels, das an einem Träger angebracht ist, zum Herstellen einer Vitrigelmembran, die an einem Träger angebracht ist;
(3) erneutes Trocknen der Vitrigelmembran, die an einem Träger angebracht ist, zum Beseitigen von freiem Wasser und Herstellen einer vitrifizierten getrockneten Vitrigelmembran, die an einem Träger angebracht ist, in dem Behälter;
(4) Rehydrieren und Entfernen der getrockneten Vitrigelmembran, die an einem Träger angebracht ist, aus dem Behälter und Befestigen der entfernten Membran an einer Folie;
(5) haftendes Befestigen der rehydrierten Vitrigelmembran, die an einem Träger angebracht und als Schicht auf der Folie angeordnet ist, an einer offenen Endfläche eines röhrenförmigen Gerüsts mit einem Klebstoff auf Urethanbasis oder mit einem doppelseitigen Klebeband oder mittels Hitzeverschweißen;
(6) Trocknen der als Schicht auf der Folie angeordneten Vitrigelmembran, die an einem Träger angebracht ist, zum Herstellen einer getrockneten Vitrigelmembran, die an einem Träger angebracht und als Schicht auf der Folie angeordnet ist;
(7) Ausformen der als Schicht auf der Folie angeordneten, getrockneten Vitrigelmembran, die an einem Träger angebracht ist, in im Wesentlichen derselben Form wie die offene Endfläche des Gerüsts; und
(8) Abnehmen der Folie von der getrockneten Vitrigelmembran, die an einem Träger angebracht ist.

17. Verfahren zur Herstellung einer Zellkultur-Doppelkammer, das den Schritt des in Verbindung Bringens des röhrenförmigen Gerüsts der Zellkultur-Einzelkammer, die unter Verwendung des Verfahrens nach einem der Ansprüche 13 bis 16 hergestellt wurde, mit einer offenen Endfläche eines weiteren röhrenförmigen Gerüsts mit derselben ebenen Querschnittsform von der Flächenseite aus, die nicht an der haftend verbundenen getrockneten Vitrigelmembran oder getrockneten Vitrigelmembran, die an einem Träger angebracht ist, haftet, und des Verbindens der beiden röhrenförmigen Gerüste miteinander und Befestigens aneinander umfasst, wobei die getrocknete Vitrigelmembran oder getrocknete Vitrigelmembran, die an einem Träger angebracht ist, dazwischen angeordnet ist.

## Revendications

1. Chambre de culture cellulaire unique comprenant une membrane de vitrigel séchée recouvrant et fixée à une surface d'extrémité ouverte d'un cadre tubulaire, dans laquelle la membrane de vitrigel séchée est fixée à la surface d'extrémité ouverte du cadre :
avec un adhésif à base d'uréthane ; ou
avec une bande double face ; ou
par thermosoudure.

2. Chambre de culture cellulaire unique selon la revendication 1, dans laquelle la membrane de vitrigel séchée présente sensiblement la même forme que la surface d'extrémité ouverte du cadre.

3. Chambre de culture cellulaire unique selon la revendication 1 ou la revendication 2, dans laquelle le cadre inclut un bouchon faisant saillie vers l'extérieur fourni sur une partie périphérique externe de la surface d'extrémité ouverte opposée à la surface d'extrémité recouverte par et fixée à la membrane de vitrigel séchée.

4. Chambre de culture cellulaire double comprenant deux cadres tubulaires joints et fixés l'un à l'autre avec une membrane de vitrigel séchée intercalée entre les surfaces d'extrémité ouvertes opposées des cadres tubulaires de manière à former une première chambre et une seconde chambre via la membrane de vitrigel séchée, dans laquelle la membrane de vitrigel séchée est fixée à une surface d'extrémité ouverte du cadre :
avec un adhésif à base d'uréthane ; ou
avec une bande double face ; ou
par thermosoudure.

5. Chambre de culture cellulaire double selon la revendication 4, dans laquelle la membrane de vitrigel séchée présente sensiblement la même forme que les surfaces d'extrémité ouvertes des cadres.

6. Chambre de culture cellulaire double selon l'une ou l'autre de la revendication 4 ou la revendication 5, dans laquelle les deux cadres sont fixés de manière adhésive l'un à l'autre depuis l'extérieur avec un matériau adhésif en forme de film autour des surfaces d'extrémité ouvertes opposées.

7. Modèle de tissu construit à partir d'une culture monocouche ou multicouche de cellules ensemencées sur la membrane de vitrigel séchée à l'intérieur de la chambre de culture cellulaire unique selon l'une quelconque des revendications 1 à 3.

8. Modèle de tissu selon la revendication 7, dans lequel le modèle de tissu est l'un quelconque parmi un modèle d'absorption transdermique de produits chimiques, un modèle de perméation cornéenne de produits chimiques, un modèle d'absorption gastro-intestinale de produits chimiques comme dans le tractus intestinal, un modèle d'absorption de produits chimiques par les voies respiratoires comme dans les poumons, un modèle de perméation vasculaire de produits chimiques, un modèle de métabolisme hépatique de produits chimiques, un modèle de filtration et d'excrétion glomérulaires rénales de produits chimiques, un modèle d'évaluation de la dermotoxicité de produits chimiques, un modèle d'évaluation de la kératotoxicité de produits chimiques, un modèle d'évaluation de la toxicité de produits chimiques pour la muqueuse buccale, un modèle d'évaluation de la neurotoxicité de produits chimiques, un modèle d'évaluation de l'hépatotoxicité de produits chimiques, un modèle d'évaluation de la néphrotoxicité de produits chimiques, un modèle d'évaluation de la toxicité de produits chimiques pour l'embryon, et un modèle d'angiogenèse ou un modèle d'infiltration de cancer pour le développement de médicaments.

9. Procédé de production de modèle de tissu comprenant l'étape d'ensemencement d'un ou de plusieurs types de cellules sur la membrane de vitrigel séchée à l'intérieur de la chambre de culture cellulaire unique selon l'une quelconque des revendications 1 à 3, et de mise en culture des cellules en une seule couche ou en plusieurs couches.

10. Modèle de tissu construit à partir d'une culture monocouche ou multicouche de cellules ensemencées sur les deux surfaces de la membrane de vitrigel séchée à l'intérieur de la chambre de culture cellulaire double selon l'une quelconque des revendications 4 à 6.

11. Modèle de tissu selon la revendication 10, dans lequel le modèle de tissu est l'un quelconque parmi un modèle d'absorption transdermique de produits chimiques, un modèle de perméation cornéenne de produits chimiques, un modèle d'absorption gastro-intestinale de produits chimiques comme dans le tractus intestinal, un modèle d'absorption de produits chimiques par les voies respiratoires comme dans les poumons, un modèle de perméation vasculaire de produits chimiques, un modèle de métabolisme hépatique de produits chimiques, un modèle de filtration et d'excrétion glomérulaires rénales de produits chimiques, un modèle d'évaluation de la dermotoxicité de produits chimiques, un modèle d'évaluation de la kératotoxicité de produits chimiques, un modèle d'évaluation de la toxicité de produits chimiques pour la muqueuse buccale, un modèle d'évaluation de la neurotoxicité de produits chimiques, un modèle d'évaluation de l'hépatotoxicité de produits chimiques, un modèle d'évaluation de la néphrotoxicité de produits chimiques, un modèle d'évaluation de la toxicité de produits chimiques pour l'embryon, et un modèle d'angiogenèse ou un modèle d'infiltration de cancer pour le développement de médicaments.

12. Procédé de production d'un modèle de tissu à l'intérieur de la chambre de culture cellulaire double selon l'une quelconque des revendications 4 à 6,
le procédé comprenant les étapes consistant à :
ensemencer des cellules sur la membrane de vitrigel à partir d'un premier côté de chambre et mettre en culture les cellules en une seule couche ou en plusieurs couches ; et
retourner la chambre de culture, et ensemencer des cellules sur la membrane de vitrigel à partir d'un second côté de chambre et mettre en culture les cellules en une seule couche ou en plusieurs couches.

13. Procédé de production d'une chambre de culture cellulaire unique comprenant les étapes consistant à :
(1) recouvrir un substrat avec un film fourni de manière amovible pour une membrane de vitrigel séchée et former un hydrogel à l'intérieur d'un moule de surface de paroi placé sur le film, ledit moule de surface de paroi étant un cadre tubulaire sans surfaces supérieure et inférieure, et permettre à une partie de l'eau libre à l'intérieur de l'hydrogel de s'écouler à travers un espace entre le substrat et le moule de surface de paroi ;
(2) retirer le moule de surface de paroi du substrat ;
(3) sécher l'hydrogel pour éliminer l'eau libre restante et produire un hydrogel sec vitrifié ;
(4) réhydrater l'hydrogel sec pour produire une membrane de vitrigel ;
(5) sécher à nouveau la membrane de vitrigel pour éliminer l'eau libre et produire une membrane de vitrigel séchée vitrifiée ;
(6) détacher la membrane de vitrigel séchée adsorbée sur le film du substrat conjointement avec le film, et fixer de manière adhésive le côté membrane de vitrigel séchée du film à une surface d'extrémité ouverte d'un cadre tubulaire avec un adhésif à base d'uréthane ou avec une bande double face ou par thermosoudure ;
(7) mettre en forme la membrane de vitrigel séchée dans sensiblement la même forme que la surface d'extrémité ouverte du cadre ; et
(8) retirer le film de la membrane de vitrigel séchée.

14. Procédé de production d'une chambre de culture cellulaire unique comprenant les étapes consistant à :
(1) former un hydrogel contenant un support dans un récipient, et sécher l'hydrogel pour éliminer l'eau libre et produire un hydrogel sec vitrifié attaché à un support ;
(2) réhydrater l'hydrogel sec attaché à un support pour produire une membrane de vitrigel attachée à un support ;
(3) sécher à nouveau la membrane de vitrigel attachée à un support pour éliminer l'eau libre et produire une membrane de vitrigel séchée vitrifiée attachée à un support dans le récipient ;
(4) réhydrater et détacher la membrane de vitrigel séchée attachée à un support du récipient, et sécher la membrane détachée maintenue entre des aimants pour produire une membrane de vitrigel séchée attachée à un support détachée du substrat ;
(5) fixer de manière adhésive la membrane de vitrigel séchée attachée à un support à une surface d'extrémité ouverte d'un cadre tubulaire avec un adhésif à base d'uréthane ou avec une bande double face ou par thermosoudure après avoir été détachée du substrat ; et
(6) mettre en forme la membrane de vitrigel séchée attachée à un support dans sensiblement la même forme que la surface d'extrémité ouverte du cadre.

15. Procédé de production d'une chambre de culture cellulaire unique comprenant les étapes consistant à :
(1) former un hydrogel contenant un support à l'intérieur d'un moule de surface de paroi placé sur un substrat, et permettre à une partie de l'eau libre à l'intérieur de l'hydrogel de s'écouler à travers un espace entre le substrat et le moule de surface de paroi ;
(2) retirer le moule de surface de paroi du substrat ;
(3) sécher l'hydrogel contenant un support pour éliminer l'eau libre restante et produire un hydrogel sec vitrifié attaché à un support ;
(4) réhydrater l'hydrogel sec attaché à un support pour produire une membrane de vitrigel attachée à un support ;
(5) sécher à nouveau la membrane de vitrigel attachée à un support pour éliminer l'eau libre et produire une membrane de vitrigel séchée vitrifiée attachée à un support sur le substrat ;
(6) réhydrater et détacher la membrane de vitrigel séchée attachée à un support du substrat, et sécher la membrane maintenue entre des aimants pour produire une membrane de vitrigel séchée attachée à un support détachée du substrat ;
(7) fixer de manière adhésive la membrane de vitrigel séchée attachée à un support à une surface d'extrémité ouverte d'un cadre tubulaire avec un adhésif à base d'uréthane ou avec une bande double face ou par thermosoudure après avoir été détachée du substrat ; et
(8) mettre en forme la membrane de vitrigel séchée attachée à un support dans sensiblement la même forme que la surface d'extrémité ouverte du cadre.

16. Procédé de production d'une chambre de culture cellulaire unique comprenant les étapes consistant à :
(1) former un hydrogel contenant un support à l'intérieur d'un récipient, et sécher l'hydrogel pour éliminer l'eau libre et produire un hydrogel sec vitrifié attaché à un support ;
(2) réhydrater l'hydrogel sec attaché à un support pour produire une membrane de vitrigel attachée à un support ;
(3) sécher à nouveau la membrane de vitrigel attachée à un support pour éliminer l'eau libre et produire une membrane de vitrigel séchée vitrifiée attachée à un support dans le récipient ;
(4) réhydrater et détacher la membrane de vitrigel séchée attachée à un support du récipient, et monter la membrane détachée sur un film ;
(5) fixer de manière adhésive la membrane de vitrigel réhydratée attachée à un support stratifiée sur le film à une surface d'extrémité ouverte d'un cadre tubulaire avec un adhésif à base d'uréthane ou avec une bande double face ou par thermosoudure ;
(6) sécher la membrane de vitrigel stratifiée attachée à un support sur le film pour produire une membrane de vitrigel séchée attachée à un support stratifiée sur le film ;
(7) mettre en forme la membrane de vitrigel séchée stratifiée attachée à un support sur le film dans sensiblement la même forme que la surface d'extrémité ouverte du cadre ; et
(8) retirer le film de la membrane de vitrigel séchée attachée à un support.

17. Procédé de production d'une chambre de culture cellulaire double comprenant l'étape consistant à mettre en contact le cadre tubulaire de la chambre de culture cellulaire unique produite en utilisant le procédé selon l'une quelconque des revendications 13 à 16 sur une surface d'extrémité ouverte d'un autre cadre tubulaire de même forme plane en coupe transversale à partir du côté de surface n'adhérant pas à la membrane de vitrigel séchée fixée de manière adhésive ou à la membrane de vitrigel séchée attachée à un support, et à joindre et fixer les deux cadres tubulaires l'un à l'autre avec la membrane de vitrigel séchée ou la membrane de vitrigel séchée attachée à un support intercalée entre eux.
